# EUROPEAN PATENT APPLICATION

(11) **EP 4 273 224 A1**
(43) Date of publication of application: **08.11.2023**
(21) Application number: 21888381.7
(22) Date of filing: 18.10.2021
(51) Int. Cl.: C12N 1/21, C12N 1/19, C12P 19/02, C12R 1/19, C12R 1/15, C12R 1/125, C12R 1/225, C12R 1/865

(54) **RECOMBINANT MICROORGANISM, PREPARATION METHOD THEREFOR, AND APPLICATION OF RECOMBINANT MICROORGANISM IN PRODUCTION OF TAGATOSE**

(30) Priority: 05.11.2020 CN 202011224203
(71) Applicant: Tiangong Biotechnology (Tianjin) Co., Ltd, Tianjin 300308 (CN)
(72) Inventor: MA, Yanhe, Tianjin 300308 (CN); SHI, Ting, Tianjin 300308 (CN); SONG, Yunhong, Tianjin 300308 (CN); ZHANG, Ting, Tianjin 300308 (CN); LI, Yunjie, Tianjin 300308 (CN)
(74) Representative: Pharma Patents International AG
(86) International application number: PCT/CN2021/124427
(87) International publication number: WO 2022/095684

(57) **Abstract**

Provided are a recombinant microorganism modified by means of genetic engineering and an application of the recombinant microorganism in production of tagatose, a preparation method for the recombinant microorganism, a tagatose production strain and a tagatose production method. According to the recombinant microorganism, the tagatose is produced by taking glucose as a substrate or taking glycerol and glucose as substrates; the efficiency of conversion and production of tagatose by means of the recombinant microorganism is high, and a multi-enzyme purification process step is not needed.

## Description

### FIELD OF THE INVENTION

The disclosure belongs to the technical field of biotechnology and genetic engineering, and in particular relates to a recombinant microorganism, a preparation method of the recombinant microorganism and its application in production of tagatose, a tagatose production strain and a tagatose production method.

### BACKGROUND OF THE INVENTION

Tagatose is a naturally occurring rare ketohexose, which is an isomer of the aldose galactose and an epimer of fructose. The sweetness of tagatose is similar to that of sucrose, and its calories are only one-third of that of sucrose, so it is called a low-calorie sweetener. Natural tagatose is mainly found in dairy products such as yogurt and milk powder. Tagatose offers a very fresh and pure sweetness, with a taste profile similar to fructose. Studies have shown that tagatose has important physiological functions such as low calorie, low glycemic index, anticaries, anti-oxidation, prebiotics, improvement of intestinal function, immune regulation, and drug precursors. It can be widely used in food, beverages, medicine, health care and other fields, and has huge economic value^{[1]}.

At present, the production methods of tagatose mainly include chemical synthesis and biotransformation. The chemical synthesis method is mainly based on galactose as raw material, using basic metal salt as a catalyst to isomerize galactose to form tagatose-metal hydroxide complex precipitation, and then using acid to neutralize to obtain tagatose. The chemical synthesis method has high energy consumption, many side reactions, and complex products, which make the separation and purification of tagatose difficult, and the addition of acids, alkalis, and metal ions is also prone to chemical pollution.

Compared with the chemical synthesis method, the biotransformation method has high conversion efficiency, strong specificity, less by-products, and simple purification steps, so it has become the main direction for the production of tagatose. The biotransformation method mainly uses galactitol or galactose as the raw material, and converts the corresponding substrate into tagatose under the catalysis of enzymes or microorganisms. However, the price of galactitol is high and the source is limited, so it is not suitable to be used as a raw material for industrial production. Using galactose as raw material, the pure tagatose is produced through isomerization, desalination, decolorization, separation, concentration, crystallization and other steps, which is the mainstream production method of tagatose. However, galactose cannot be completely converted into tagatose, and the final product is a mixture of galactose and tagatose, which requires complex separation processes to separate pure tagatose, which increases the difficulty and cost of the process; meanwhile the high price of galactose leads to the high cost of tagatose production^{[2-4]}. Therefore, how to reduce the process cost and difficulty of tagatose production while ensuring the conversion efficiency of tagatose is an important problem to be solved urgently.

Patent Document 1 discloses a method for production of tagatose by multi-enzyme catalytic conversion, wherein the catalyzed enzymes include fructokinase, 6-phosphate tagatose epimerase, and 6-phosphate tagatose phosphatase, which convert fructose to tagatose. However, the process of converting fructose into fructose-6-phosphate by fructokinase requires additional addition of ATP to phosphorylate fructose as a substrate, and the addition of expensive ATP leads to high costs of tagatose production, which is not industrially viable.

Patent Document 2 discloses a hexuronate C4-epimerase variant with improved hexuronate C4-epimerase conversion activity, using the hexuronate C4-epimerase variant to convert fructose into tagatose. However, the modified hexuronate C4-epimerase variant still has low activity, resulting in low conversion efficiency of tagatose, which cannot meet the needs of industrial production.

Patent Document 3 discloses a tagatose-6-phosphate phosphatase, which can be applied to the conversion and production of tagatose using starch, maltodextrin, sucrose, etc. as substrates. However, the tagatose-6-phosphate phosphatase still has the problems of low enzyme activity and low utilization value in actual industrial production.

Patent Document 4 discloses a method for preparation of targose, in which a multi-enzyme molecular machine is established to carry out a multi-enzyme-catalyzed reaction with starch or a starch derivative as substrate, containing a-glucan phosphatase, phosphoglucomutase, glucose phosphoisomerase, 6-phosphate tagatose epimerase, 6-phosphate tagatose phosphatase and inorganic phosphate ions, to obtain targose. Although the preparation method of Patent Document 4 improves the conversion rate of raw materials and the yield of tagatose, after the synthesis of tagatose, tagatose needs to be purified from a mixture of various enzymes, which increases the steps and difficulty of the purification process.

### Reference Documents:

[1] Oh D-K: Tagatose: properties, applications, and biotechnological processes. App. Microbiol. Biotechnol. 2007, 76:1-8.
(2] Rhimi M, Aghajari N, Juy M, Chouayekh H, Maguin E, Haser R, Bejar S: Rational design of Bacillus stearothermophilus US 1001-arabinose isomerase: Potential applications for d-tagatose production. Biochim. 2009, 91:650-653.
[3] Oh H-J, Kim H-J, Oh D-K: Increase in d-tagatose production rate by site-directed mutagenesis of 1-arabinose isomerase from Geobacillus thermodenitrificans. Biotechnol. Lett. 2006, 28:145-149.
[4] Bosshart A, Hee CS, Bechtold M, Schirmer T, Panke S: Directed divergent evolution of a thermostable D-tagatose epimerase towards improved activity for two hexose substrates. ChemBioChem 2015,16:592-601.

Patent Document 1: WO2015016544A1
Patent Document 2: CN109415715A
Patent Document 3: WO2018004310A1
Patent Document 4: CN106399427A

### SUMMARY OF THE INVENTION

### Problems to be solved by the invention

In view of the technical problems existing in the prior art, for example, the existing tagatose production method has the defects of high raw material cost, low conversion rate, and the need for multiple enzyme purification steps, which complicates the tagatose production process, to this end, the present disclosure provides a recombinant microorganism that can convert and produce tagatose by using glycerol and glucose as substrates, and has the advantages of high conversion efficiency, high environmental friendliness, no need for multi-enzyme purification, and low production cost, and is suitable for industrial production of tagatose.

### Solutions to problems

(1) a recombinant microorganism, wherein the recombinant microorganism has at least one of the following characteristics (a)-(c), as compared to a wild-type microorganism.
   (a) reduced or lost protein activity of glucose-specific transfer protein of phosphotransferase system and/or expression level of its coding gene;
   (b) enhanced enzyme activity of tagatose-6-phosphate epimerase and/or expression level of its coding gene;
   (c) enhanced enzyme activity of tagatose-6-phosphate phosphatase and/or expression level of its coding gene.
(2) The recombinant microorganism according to (1), wherein the glucose-specific transfer protein is selected from the group shown in any one the following (a₁)-(a₃):
   (a₁) a polypeptide comprising an amino acid sequence as set forth in SEQ ID NO: 1 or SEQ ID NO: 3 and having glucose-specific transfer protein activity;
   (a₂) a polypeptide having an amino acid sequence obtained by substituting, repeating, deleting or adding one or more amino acids to the amino acid sequence as set forth in SEQ ID NO: 1 or SEQ ID NO: 3, and having glucose-specific transfer protein activity;
   (a₃) a polypeptide encoded by a nucleotide sequence as set forth in SEQ ID NO: 2 or SEQ ID NO: 4.
   optionally, the tagatose-6-phosphate epimerase is selected from the group shown in any of the following (b₁)-(b₃):
   (b₁) a polypeptide comprising an amino acid sequence as set forth in SEQ ID NO: 5 and having tagatose-6-phosphate epimerase activity;
   (b₂) a polypeptide having an amino acid sequence obtained by substituting, repeating, deleting or adding one or more amino acids to the amino acid sequence as set forth in SEQ ID NO: 5, and having tagatose-6-phosphate epimerase activity;
   (b₃) a polypeptide encoded by a nucleotide sequence as set forth in SEQ ID NO: 6;
   optionally, the tagatose-6-phosphate phosphatase is selected from the group shown in any of the following (c₁)-(c₃):
   (ci) a polypeptide comprising an amino acid sequence as set forth in SEQ ID NO: 7 or SEQ ID NO: 54 and having tagatose-6-phosphate phosphatase activity;
   (c₂) a polypeptide having an amino acid sequence obtained by substituting, repeating, deleting or adding one or more amino acids to the amino acid sequence as set forth in SEQ ID NO: 7 or SEQ ID NO: 54, and having tagatose-6-phosphate phosphatase activity;
   (c₃) a polypeptide encoded by a nucleotide sequence as set forth in SEQ ID NO: 8 or SEQ ID NO: 55.
(3) The recombinant microorganism according to (1) or (2), wherein, as compared to the wild-type microorganism, the recombinant microorganism further has at least one of the following characteristics (d)-(j):
   (d) enhanced enzyme activity of glucokinase and/or expression level of its coding gene;
   (e) enhanced enzyme activity of glucose-6-phosphate isomerase and/or expression level of its coding gene;
   (f) reduced or lost enzyme activity of fructose-6-phosphate kinase and/or expression level of its coding gene;
   (g) reduced or lost enzyme activity of pyruvate kinase and/or expression level of its coding gene;
   (h) reduced or lost enzyme activity of phosphoglucomutase and/or expression level of its coding gene;
   (i) reduced or lost enzyme activity of glucose-6-phosphate dehydrogenase and/or expression level of its coding gene;
   (j) reduced or lost enzyme activity of HPr kinase and/or expression level of its coding gene.
(4) The recombinant microorganism according to (3), wherein the glucokinase is selected from the group shown in any one of the following (d₁)-(d₃):
   (d₁) a polypeptide comprising an amino acid sequence as set forth in SEQ ID NO: 9 or SEQ ID NO: 11 and having glucokinase activity;
   (d₂) a polypeptide having an amino acid sequence obtained by substituting, repeating, deleting or adding one or more amino acids to the amino acid sequence as set forth in SEQ ID NO:9 or SEQ ID NO: 11, and having glucokinase activity;
   (d₃) a polypeptide encoded by a nucleotide sequence as set forth in SEQ ID NO: 10 or SEQ ID NO: 12;
   optionally, the glucose-6-phosphate isomerase is selected from the group shown in any of the following (e₁)-(e₃):
   (e₁) a polypeptide comprising an amino acid sequence as set forth in in SEQ ID NO: 13 or SEQ ID NO: 15 and having glucose-6-phosphate isomerase activity;
   (ez) a polypeptide having an amino acid sequence obtained by substituting, repeating, deleting or adding one or more amino acids to the amino acid sequence as set forth in SEQ ID NO: 13 or SEQ ID NO: 15, and having glucose-6-phosphate isomerase activity;
   (e₃) a polypeptide encoded by a nucleotide sequence as set forth in in SEQ ID NO: 14 or SEQ ID NO: 16.
(5) The recombinant microorganism according to any one of (1)-(4), wherein the recombinant microorganism is derived from *Escherichia coli, Corynebacterium glutamicum, Bacillus subtilis, Lactobacillus* or *Saccharomyces cerevisiae.*
(6) A preparation method of the recombinant microorganism according to any one of claims 1-5, wherein the preparation method comprises following steps:
   a step of knocking out or knocking down a gene encoding glucose-specific transfer protein of phosphotransferase system in wild-type microorganism;
   a step of introducing a recombinant expression vector expressing tagatose-6-phosphate epimerase and tagatose-6-phosphate phosphatase into the recombinant microorganism, or a step of introducing recombinant expression vectors respectively expressing tagatose-6-phosphate epimerase and tagatose-6-phosphate phosphatase into the recombinant microorganism.
(7) The preparation method according to (6), wherein the preparation method further comprises at least one of following steps:
   a step of enhancing expression level of a gene encoding glucokinase in the recombinant microorganism;
   a step of enhancing expression level of a gene encoding glucose-6-phosphate isomerase in the recombinant microorganism;
   a step of knocking out or knocking down a gene encoding fructose-6-phosphate kinase in the recombinant microorganism;
   a step of knocking out or knocking down a gene encoding pyruvate kinase in the recombinant microorganism;
   a step of knocking out or knocking down a gene encoding phosphoglucomutase in the recombinant microorganism;
   a step of knocking out or knocking down a gene encoding glucose-6-phosphate dehydrogenase in the recombinant microorganism;
   a step of knocking out or knocking down a gene encoding HPr kinase in the recombinant microorganism.
(8) Use of the recombinant microorganism according to any one of (1)-(5), or a recombinant microorganism prepared by the method according to (6) or (7) in production of tagatose.
(9) A tagatose production strain, wherein the tagatose production strain is the recombinant microorganism according to any one of (1)-(5), or a recombinant microorganism prepared by the method according to (6) or (7).
(10) The tagatose production strain according to (9), wherein the tagatose production strain uses glucose or glucose and glycerol as substrates;
   preferably, the tagatose production strain is derived from *Escherichia coli, Corynebacterium glutamicum, Bacillus subtilis, Lactobacillus* or *Saccharomyces cerevisiae.*
(11) A tagatose production method, comprising a step of utilizing the recombinant microorganism according to any one of (1)-(5), a recombinant microorganism prepared by the method according to (6) or (7), or the tagatose production strain according to (9) or (10) to carry out a fermentation reaction with glucose or glucose and glycerol as substrates.
(12) The tagatose production method according to (11), further comprising a step of separating tagatose from fermentation reaction liquid after the fermentation reaction is finished.

### Technical effects of the invention

In one embodiment, the present disclosure provides a recombinant microorganism, its reduced or lost activity of glucose-specific transfer protein of phosphotransferase system can reduce or eliminate the inhibitory effect of glucose on use of glycerol substrate by the recombinant microorganism, enabling the recombinant microorganism to produce tagatose using glycerol and glucose as substrates. The raw material price is low, the source is wide, the efficiency of conversion into tagatose is high, and multi-enzyme purification process steps are not required, the production cost and environmental pollution are reduced, and the method has important industrial application value.

In one embodiment, the preparation method of the recombinant microorganism provided in the present disclosure has a simple preparation process and is easy to implement, and can obtain a recombinant microorganism that uses glucose and glycerol as substrates to produce tagatose.

In one embodiment, the tagatose production strain provided by the present disclosure uses glycerol and glucose as substrates, wherein glycerol is used as a carbon source for intra-strain metabolism to supply the growth of the strain, so that it uses glucose to carry out the tagatose synthesis pathway under conditions suitable for growth, and obtains tagatose with increased yield.

In one embodiment, the tagatose production method provided by the present disclosure uses a recombinant microorganism or a tagatose production strain for fermentation production, which has the advantages of high conversion efficiency of raw materials, high yield of tagatose, simplified process and reduced cost, and is suitable for industrial scale production of tagatose.

### BRIEFT DESCRIPTION OF THE DRAWINGS

Fig. 1 shows a schematic diagram of the metabolic process of recombinant microorganism to produce tagatose with glucose and glycerol as substrates.
Figs. 2A-2D show maps of vectors pTKSCS, pTKRed, pACYCDuet, pETDuet, respectively, wherein Fig. 2A is the vector map of pTKSCS, Fig. 2B is the vector map of pTKRed, Fig. 2C is the vector map of pACYCDuet, and Fig. 2D is the vector map of pETDuet.
Fig. 3A-Fig. 3B respectively show maps of the vectors pSS and pWB980, wherein Fig. 3A is the vector map of pSS, and Fig. 3B is the vector map of pWB980.
Fig. 4 shows analysis results of high-performance liquid chromatography of tagatose produced by fermentation of glycerol and glucose by tagatose producing *Escherichia coli* engineered strain YH6-2.
Fig. 5 shows analysis results of high-performance liquid chromatography of tagatose produced by fermentation of glycerol and glucose by tagatose producing *Bacillus subtilis* engineered strain YJ14-1.

### DETAILED DESCRIPTION OF THE INVENTION

### Definitions

When used in conjunction with the term "comprising" in the claims and/or specification, the words "a" or "an" may mean "one", but may also mean "one or more", "at least one" and "one or more".

As used in the claims and specification, the words "including", "having", "comprising" or "containing" mean inclusive or open-ended and do not exclude additional, non-recited elements or means step.

Throughout this application, the term "about" means that a value includes the standard deviation of error of the apparatus or method used to determine the value.

Although the disclosure supports the definition of the term "or" as an alternative only and "and/or", unless expressly stated to be only alternative or mutually exclusive between alternatives, the term "or" in the claims " means "and/or".

When used in claims or specification, the selected/optional/preferred "range of values" includes both the numerical endpoints at either end of the range and all natural numbers covered in the middle of the numerical endpoints relative to the aforementioned numerical endpoints.

As used in the present disclosure, although other organic or inorganic catalysts may be used, the term "converting" refers to a chemical conversion from one molecule to another catalyzed primarily by one or more polypeptides (enzymes); it may also refer to the ratio (in %) between the molar amount of the desired product and the molar amount of the limited substrate.

As used in the present disclosure, the terms "polypeptide", "enzyme", "polypeptide or enzyme", and "polypeptide/enzyme" have the same meaning and are interchangeable for the purposes of the present disclosure. The foregoing term refers to a polymer consisting of a number of amino acids via peptide bonds which may or may not contain modifications such as phosphate groups and formyl groups.

As used in the present disclosure, the term "glucose-specific transfer protein of phosphotransferase system" refers to a glucose-specific transfer protein involved in the phosphotransferase system (PTS). The PTS system is widely found in bacteria, fungi and some archaea, but not in plants and animals. The PTS system mediates the regulation of carbon metabolism in organisms, and there is a phenomenon of inducer exclusion. Inducer exclusion means that when a preferred carbon source (such as PTS carbon source-glucose) is transported and metabolized, it will inhibit the uptake and utilization of other non-preferred carbon sources. Taking *Escherichia coli* as an example, when glucose and other carbon sources (such as lactose, maltose, glycerol, etc.) are present in the medium at the same time, glucose is preferentially utilized, resulting in cascade transfer of phosphate groups between glucose-specific transfer proteins of the PTS system, and increased dephosphorylation of glucose-specific transfer proteins. Unphosphorylated glucose-specific transfer proteins inhibit the transport and phosphorylation of carbon sources such as lactose, maltose, and glycerol by binding to the respective transporters or kinases (such as LacY, MalK, GlpK).

As used in the present disclosure, the term "tagatose-6-phosphate epimerase", also known as Tagatose-6-phosphate 4-epimeras (T6PE), can catalyze the interconversion of fructose-6-phosphate and tagatose-6-phosphate. In some embodiments, the tagatose-6-phosphate epimerase of the present disclosure is derived from *Agrobacterium tumefaciens.* In a specific embodiment, the tagatose-6-phosphate epimerase of the present disclosure is derived from the strain *Agrobacterium tumefaciens str. C58.*

As used in the present disclosure, the term "tagatose-6-phosphate phosphatase", also known as 6-phosphate tagatose phosphatase (T6PP), can catalyze the conversion of tagatose-6-phosphate to tagatose. In some embodiments, the tagatose-6-phosphate phosphatase of the present disclosure is derived from the genus *Archaeoglobus fulgidus, Archaeoglobus profundus.*

As used in the present disclosure, the term " glucokinase (glk)" is a type of hexokinase isoenzyme, which is involved in the process of phosphorylation of glucose to produce glucose 6-phosphate, (G6P). The process consumes one ATP, converts it into ADP, and requires the participation of Mg²⁺. In some embodiments, the glucokinase of the present disclosure is derived from *Escherichia coli;* in other embodiments, the glucokinase can also be derived from strains such as *Corynebacterium glutamicum, Bacillus subtilis, Lactobacillus* or *Saccharomyces cerevisiae* that use glucose as a substrate to undergo sugar metabolism.

As used in the present disclosure, the term "glucose-6-phosphate isomerase", also known as 6-phosphate glucose isomerase (G6PI, pgi), exists in the cytoplasm and extracellular fluid. Its main function is to catalyze the interconversion between D-glucose-6-phosphate and D-fructose-6-phosphate. It is an important enzyme for glycolysis and gluconeogenesis. In addition to enzyme activity, it also has cell and growth factors activity. In some embodiments, the glucose-6-phosphate isomerase of the present disclosure is derived from *Escherichia coli;* in some embodiments, the glucose-6-phosphate isomerase of the present disclosure is derived from *Bacillus subtilis;* in some other embodiments, the glucose-6-phosphate isomerase can also be derived from strains such as *Corynebacterium glutamicum, Lactobacillus* or *Saccharomyces cerevisiae* that use glucose as a substrate to undergo sugar metabolism.

As used in the present disclosure, the term "glucose phosphomutase", also known as phosphoglucomutase (pgm), can catalyze the interconversion of glucose-1-phosphate and glucose-6-phosphate, and plays an important role in glucose metabolism. In some embodiments, the phosphoglucomutase of the present disclosure is derived from *Escherichia coli*; in some embodiments, the phosphoglucomutase of the present disclosure is derived from *Bacillus subtilis;* in some other embodiments, the phosphoglucomutase can also be derived from strains such as *Corynebacterium glutamicum, Lactobacillus* or *Saccharomyces cerevisiae* that use glucose as a substrate to undergo sugar metabolism.

As used in the present disclosure, the term "glucose-6-phosphate dehydrogenase" is an oxidoreductase that takes NAD+ or NADP+ as an acceptor and acts on a donor CH-OH group. This enzyme can catalyze the following enzymatic reaction: D-glucose-6-phosphate + NADP⁺ = D-glucono-1,5-lactone-6-phosphate + NADPH + H⁺. Glucose-6-phosphate dehydrogenase is mainly involved in the pentose phosphate pathway and can also slowly act on other sugars such as β-D-glucose. In some embodiments, the glucose-6-phosphate dehydrogenase of the present disclosure is derived from *Escherichia coli;* in some embodiments, the glucose-6-phosphate dehydrogenase of the present disclosure is derived from *Bacillus subtilis;* in some other embodiments, the glucose-6-phosphate dehydrogenase can also be derived from strains such as *Corynebacterium glutamicum, Lactobacillus* or *Saccharomyces cerevisiae* that use glucose as a substrate to undergo sugar metabolism.

As used in the present disclosure, the term "fructose-6-phosphate kinase", also known as 6-phosphofructokinase (phosphofructokinase, pfk) is a class of kinases that can act on fructose-6-phosphate, which is acted by phosphofructokinase 1 (pfk-1) to obtain fructose-1,6-diphosphate, or acted by phosphofructokinase 2 (pfk-2) to obtain fructose-2,6-diphosphate. In some embodiments, the fructose-6-phosphate kinase of the present disclosure is derived from *Escherichia coli,* including pfkA and pfkB; in some embodiments, the fructose-6-phosphate kinase of the present disclosure is derived from *Bacillus subtilis,* including pfkA; in some other embodiments, the glucokinase can also be derived from strains such as *Corynebacterium glutamicum, Lactobacillus* or *Saccharomyces cerevisiae* that undergo sugar metabolism.

As used in the present disclosure, the term "pyruvate kinase (PK)", also known as pyruvate phosphotransferase, phosphopyruvate kinase, catalyzes the transfer of a phosphate group from phosphoenolpyruvate (PEP) to ADP, generating a molecule of pyruvate and a molecule of ATP. Pyruvate kinase converts phosphoenolpyruvate and ADP into ATP and pyruvate and is one of the main rate-limiting enzymes in glycolysis. In some embodiments, the pyruvate kinase of the present disclosure is derived from *Escherichia coli,* including pykA and pykF. In some other embodiments, the pyruvate kinase may also be derived from strains such as *Corynebacterium glutamicum, Bacillus subtilis, Lactobacillus* or *Saccharomyces cerevisiae.*

As used in the present disclosure, the term "HPr kinase" (HPrK) refers to a specific transfer protein element involved in the phosphotransferase system (PTS). In the presence of sugars transported by PTS such as glucose, fructose-1,6-bisphosphate will promote Hpr kinase (HPrK) to phosphorylate serine 46 of HPr to generate phosphorylated product P-Ser-HPr. The phosphorylated product P-Ser-HPr further binds to the catabolite control protein (CcpA), and the binding product interacts with the catabolite response element (ere) region in front of the Hpr kinase coding gene to exert a metabolite repression effect, making the cell unable to transport glycerol into the cell for metabolism.

As used in the present disclosure, the terms "enzyme activity" and "protein activity" are also expressed as "specific activity", which have the same meaning in the present disclosure and can be used interchangeably. It refers to the enzyme activity (U/mg) and protein activity (U/mg) per milligram of polypeptide (enzyme, protein).

The term "expression" in the present disclosure includes any step involved in RNA production and protein production, including but not limited to: transcription, post-transcriptional modification, translation, post-translational modification, and secretion.

The term "coding gene" in the present disclosure refers to a synthetic DNA molecule that can guide proteins through certain rules. The process of protein-coding gene guiding protein synthesis generally includes the transcription process using double-stranded DNA as a template and the translation process using mRNA as a template. The coding gene contains CDS sequence (Coding Sequence), which can guide the production of mRNA encoding protein. The coding genes involved in the present disclosure include the coding genes of proteins and enzymes such as glucose-specific transfer protein of phosphotransferase system, tagatose-6-phosphate epimerase, tagatose-6-phosphate phosphatase, glucokinase, glucose-6-phosphate isomerase, fructose-6-phosphokinase, pyruvate kinase, phosphoglucomutase, glucose-6-phosphate dehydrogenase, HPr kinase.

As used in the present disclosure, the term "wild-type" refers to an object that can be found in nature. For example, a polypeptide, polynucleotide sequence, or microorganism that can be isolated from a source in nature and that has not been intentionally modified by humans in the laboratory is naturally occurring. As used in the present disclosure, "naturally occurring" and "wild-type" are synonyms.

As used in the present disclosure, the term "microorganism" is a general term for tiny organisms that are difficult to see with the naked eye, including bacteria, fungi, and the like. Due to the large surface area to volume ratio of microorganisms, they can quickly exchange substances with the external environment and produce metabolites. The microorganisms in the present disclosure specifically refer to fermenting microorganisms that can be fermented and cultivated to produce metabolites such as carbohydrates, lipids, amino acids, and nucleotides.

As used in the present disclosure, the term "recombinant microorganism" refers to a modified microorganism obtained by recombination through genetic engineering methods. Embodiments include but are not limited to introducing recombinant genes, knocking out and knocking down endogenous genes of microorganisms, and other operations. Wherein, the term "recombinant gene" refers to a gene that does not occur in nature, and the recombinant gene includes a protein coding sequence operably linked to an expression control sequence. Embodiments include, but are not limited to, exogenous genes introduced into a microorganism, endogenous protein coding sequences operably linked to heterologous promoters, and genes with modified protein coding sequences. The recombinant gene is stored on the genome of the microorganism, on a plasmid in the microorganism, or on a phage in the microorganism.

In some embodiments, a recombinant microorganism with reduced or lost protein activity, reduced or lost expression level of protein-coding gene, reduced or lost enzyme activity, reduced or lost expression level of enzyme-coding gene comprises recombinant microorganisms modified by the following genetic engineering methods: introducing a weak promoter, a weak ribosome binding site into cells of microorganisms, knocking out or knocking down genes encoding proteins and enzymes, and inserting random fragments into the genes encoding proteins and enzymes to lose the activitives of proteins and enzymes.

In some embodiments, a recombinant microorganism with enhanced protein activity, enhanced expression level of protein-coding gene, enhanced enzyme activity, enhanced expression level of enzyme-coding gene comprises recombinant microorganisms modified by the following genetic engineering methods: introducing a strong promoter, a strong ribosomal binding site, introducing a non-integrated recombinant expression vector for a protein or an enzyme, and a chromosomally integrated recombinant expression vector for a protein or an enzyme into cells of microorganisms.

In some embodiments, the recombinant microorganism of the present disclosure is a recombinant strain transformed by genetic engineering methods using *Escherichia coli* as a wild-type microorganism; in some embodiments, the recombinant microorganism of the present disclosure is a recombinant strain transformed by genetic engineering methods using *Bacillus subtilis* as a wild-type microorganism; in some other embodiments, the recombinant microorganism of the present disclosure may also be a recombinant strain modified by genetic engineering methods using fermenting strains such as *Corynebacterium glutamicum, Lactobacillus* or *Saccharomyces cerevisiae* as wild-type microorganisms.

As used in the present disclosure, the term "operably linked" refers to a configuration in which a regulatory sequence is positioned relative to the coding sequence of a polynucleotide such that the regulatory sequence directs the expression of the coding sequence. Exemplarily, the regulatory sequence may be selected from sequences encoded by promoters and/or enhancers.

As used in the present disclosure, the term "endogenous" refers to a polynucleotide, polypeptide or other compound that is naturally expressed or produced within an organism or cell. That is, an endogenous polynucleotide, polypeptide or other compound is not exogenous. For example, an "endogenous" polynucleotide or polypeptide is present in the cell when the cell was originally isolated from nature.

As used in the present disclosure, the term "exogenous" refers to any polynucleotide or polypeptide that is naturally found or expressed in the particular cell or organism in which expression is desired. An exogenous polynucleotide, polypeptide or other compound is not endogenous.

As used in the present disclosure, the term "amino acid mutation" or "nucleotide mutation" includes "substitution, repetition, deletion or addition of one or more amino acids or nucleotides". In the present disclosure, the term "mutation" refers to a change in nucleotide sequence or amino acid sequence. In a specific embodiment, the term "mutation" refers to "substitution".

In an embodiment, the "mutations" of the present disclosure may be selected from "conservative mutation". In the present disclosure, the term "conservative mutation" refers to a mutation that can normally maintain the function of a protein. A representative example of a conservative mutation is a conservative substitution.

As used in the present disclosure, the term "conservative substitution" involves the replacement of an amino acid residue with an amino acid residue having a similar side chain. Families of amino acid residues with similar side chains have been defined in the art and include those with basic side chains (e.g. lysine, arginine and histidine), acidic side chains (e.g. aspartic acid and glutamic acid), uncharged polar side chains (e.g. glycine, asparagine, glutamine, serine, threonine, tyrosine, and cysteine), non-polar side chains (e.g. alanine, valine, leucine, isoleucine, proline, phenylalanine, methionine and tryptophan), β-branched chains (e.g. threonine, valine and isoleucine) and aromatic side chains (e.g. tyrosine, phenylalanine, tryptophan and histidine). As used in the present disclosure, a "conservative substitution" typically involves the exchange of an amino acid at one or more sites in a protein. Such substitutions can be conservative. In addition to substitutions that are regarded as conservative substitutions, conservative mutations also include naturally occurring mutations that arise from individual differences in the origin of genes, strains, species, etc.

As used in the present disclosure, the term "polynucleotide" refers to a polymer composed of nucleotides. A polynucleotide may be in the form of an individual fragment or an integral part of a larger nucleotide sequence structure which is a nucleotide sequence derived from a nucleotide sequence that has been isolated at least once in quantity or concentration and is capable of being identified, manipulated, and recovered from the sequence and its components by standard molecular biology methods (e.g., using cloning vectors). When a nucleotide sequence is represented by a DNA sequence (i.e., A, T, G, C), this also includes an RNA sequence (ie A, U, G, C), where "U" replaces "T". In other words, "polynucleotide" refers to a polymer of nucleotides removed from other nucleotides (individual fragments or entire fragments), or which may be a building block or component of a larger nucleotide structure, such as the expression vector or polycistronic sequence. Polynucleotides include DNA, RNA and cDNA sequences. "Recombinant polynucleotide" is a kind of "polynucleotide".

As used in the present disclosure, the term "vector" refers to a DNA construct containing DNA sequences operably linked to appropriate control sequences to express a gene of interest in a suitable host. "Recombinant expression vector"refers to a DNA construct used to express, for example, a polynucleotide encoding a desired polypeptide. Recombinant expression vectors may include, for example, i) a collection of genetic elements that have a regulatory effect on gene expression, such as promoters and enhancers; ii) a structural or coding sequence that is transcribed into mRNA and translated into protein; and iii) appropriate transcription and translation initiation and termination sequences of the transcriptional subunit. Recombinant expression vectors are constructed in any suitable manner. The nature of the vector is not critical, and any vector may be used, including plasmids, viruses, phage and transposons. Possible vectors for use in the present disclosure include, but are not limited to, chromosomal, non-chromosomal, and synthetic DNA sequences, such as bacterial plasmids, phage DNA, yeast plasmids, and vectors derived from combinations of plasmids and phage DNA, DNA from viruses such as vaccinia, adenovirus, fowlpox, baculovirus, SV40, and pseudorabies.

As used in the present disclosure, the term "transduction" has the meaning commonly understood by those skilled in the art, i.e., the process of introducing exogenous DNA into a host. The transformation method includes any method for introducing nucleic acid into cells, and these methods include but are not limited to electroporation, calcium phosphate (CaPO₄) precipitation, calcium chloride (CaCh) precipitation, microinjection, polyethylene glycol (PEG) method, DEAE-dextran method, cationic liposome method and lithium acetate-DMSO method.

As used in the present disclosure, the term "tagatose yield" has the meaning commonly understood by those skilled in the art, that is, the percentage of substrate consumed to produce tagatose in the total substrate. In the present disclosure, "tagatose yield" and "substrate conversion rate" may be used interchangeably.

The cultivation of the recombinant microorganisms of the present disclosure can be carried out according to conventional methods in the art, including but not limited to well plate culture, shake flask culture, batch culture, continuous culture and fed-batch culture, and various culture conditions such as temperature, time and pH of the culture medium can be adjusted as appropriate.

Unless otherwise defined or clearly indicated by the context, all technical and scientific terms in the present disclosure have the same meaning as commonly understood by one of ordinary skill in the art to which the present disclosure belongs.

### Tagatose production strain

In a technical solution, the present disclosure adopts a method of genetically modifying wild-type microorganism to obtain a recombinant microorganism, which in turn produce tagatose.

In a specific embodiment, the recombinant microorganism used in the present disclosure is derived from *Escherichia coli, Corynebacterium glutamicum, Bacillus subtilis, Lactobacillus* or *Saccharomyces cerevisiae.*

In a specific embodiment, the present disclosure uses *Escherichia coli* and *Bacillus subtilis* as wild-type microorganisms, and performs genetic engineering on *Escherichia coli* and *Bacillus subtilis* respectively.

In a specific embodiment, the present disclosure eliminates enzyme activity of glucose-specific transfer protein of the PTS system, for example, by knocking out the gene encoding the glucose-specific transfer protein of the PTS system, or inserting a random fragment into the gene encoding the glucose-specific transfer protein of the PTS system to inactivate the enzyme activity of the glucose-specific transfer protein of the PTS system.

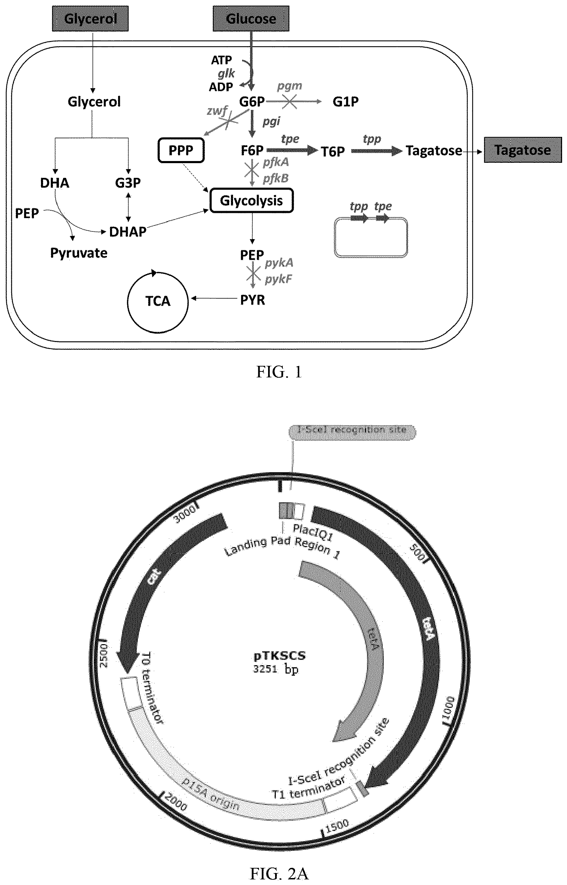

In a specific embodiment, the purpose of eliminating the glucose-specific transfer protein of the PTS system in the present disclosure is to eliminate the inhibitory effect of glucose on the utilization of glycerol substrate. Recombinant strains of *Escherichia coli* or *Bacillus subtilis* use glycerol and glucose as substrates at the same time, in which glycerol is used as a carbon source to enter the cell metabolism to supply the growth of the strain, and under conditions capable of growth, the strain can use glucose as a carbon source to enter the intracellular tagatose synthesis pathway to synthesize tagatose (Fig. 1).

In a specific embodiment, the present disclosure enhances the enzyme activity of glucokinase and glucose-6-phosphate isomerase, for example, by introducing a strong promoter or adopting a plasmid free expression form to increase the expression intensity of genes encoding glucokinase and glucose-6-phosphate isomerase, or by integrating genes encoding glucokinase and glucose-6-phosphate isomerase with higher enzyme activity from other species sources using chromosomal integration.

In a specific embodiment, the present disclosure eliminates the enzyme activity of fructose-6-phosphate kinase, for example by knocking out the gene encoding fructose-6-phosphate kinase, or inserting a random fragment into the gene encoding fructose-6-phosphate kinase to inactivate the enzyme activity of fructose-6-phosphate kinase.

In a specific embodiment, the present disclosure enhances the enzyme activity of tagatose-6-phosphate epimerase and tagatose-6-phosphate phosphatase, for example by employing a strong promoter, a strong ribosome binding site, chromosomal integration or plasmid free expression form to enhance the expression intensity of genes enconding tagatose-6-phosphate epimerase and tagatose-6-phosphate phosphatase.

In a specific embodiment, the present disclosure eliminates the enzyme activity of pyruvate kinase, for example, by knocking out the gene encoding pyruvate kinase, or inserting a random fragment into the gene encoding pyruvate kinase to inactive the enzyme activity of pyruvate kinase.

In the glycerol metabolic pathway i.e., the step where glycerol enters the cell to produce glyceric acid-3-phosphate (G3P), phosphoenolpyruvate is provided by PEP with a high-energy phosphate group that drives glycerol to G3P, thereby accelerating the ability to metabolize glycerol, a step that is useful for glycerol-dependent growth of the strain.

In a specific embodiment, the present disclosure eliminates the enzyme activity of phosphoglucomutase, for example, by knocking out the gene encoding phosphoglucomutase, or inserting a random fragment into the gene encoding phosphoglucomutase to inactive the enzyme activity of phosphoglucomutase.

In a specific embodiment, the present disclosure reduces the enzyme activity of glucose-6-phosphate dehydrogenase, for example, by knocking out the gene encoding glucose-6-phosphate dehydrogenase, or by inserting a random fragment into the gene encoding glucose-6-phosphate dehydrogenase to inactivate the enzyme activity of glucose-6-phosphate dehydrogenase; or by reducing the transcriptional expression level of the gene encoding glucose-6-phosphate dehydrogenase through a weak promoter or a weak ribosome binding site, thereby reducing the enzyme activity of glucose-6-phosphate dehydrogenase.

In a specific embodiment, the present disclosure eliminates the enzyme activity of HPr kinase, for example, by knocking out the gene encoding HPr kinase, or inserting a random fragment into the gene encoding HPr kinase to inactive the enzyme activity of HPr kinase. The purpose of eliminating HPr kinase is to eliminate the inhibitory effect of glucose on glycerol substrate utilization.

### Construction method of an engineered strain of Escherichia coli for producing tagatose

In one technical solution, the present disclosure provides a construction method of an engineered strain of *Escherichia coli* for producing tagatose.

In a specific embodiment, the present disclosure designs primers containing the upstream and downstream homology arms of the PTS system glucose-specific transfer protein coding gene *ptsG* of *Escherichia coli.* The Scel-tet-Scel fragment on the vector pTKSCS (Fig. 2A) is amplified by PCR technology, and the homologous recombination fragment is transformed into *Escherichia coli* MG1655 (DE3), and the PTS system glucose-specific transfer protein coding gene *ptsG* is knocking out by λ-Red homologous recombination technology to obtain recombinant strain YH1.

In a specific embodiment, the present disclosure amplifies the glucokinase coding gene *glcK* derived from *Escherichia coli* and the glucose-6-phosphate isomerase coding gene *pgi* derived from *Escherichia coli,* and constructs them into the expression vector pACYCDuet to obtain the recombinant expression vector pACYCDuet-glcK-pgi, and the recombinant expression pACYCDuet-glcK-pgi is transformed into *Escherichia coli* YH1 to obtain recombinant strain YH2.

In a specific embodiment, the present disclosure designs primers containing the upstream and downstream homology arms of the fructose-6-phosphate kinase coding gene *pfkA* of *Escherichia coli.* The SceI-tet-SceI fragment on the vector pTKSCS (Fig. 2A) is amplified by PCR technology, the homologous recombination fragment is transformed into the recombinant strain YH2, and the fructose-6-phosphate kinase coding gene *pfkA* is knocked out by λ-Red homologous recombination technology to obtain the recombinant strain YH3. The recombinant expression vector pACYCDuet-glcK-pgi is transformed into *Escherichia coli* YH3 to obtain recombinant strain YH3-1.

In a specific embodiment, the present disclosure designs primers containing the upstream and downstream homology arms of the pyruvate kinase coding gene *pykF* of *Escherichia coli.* The SceI-tet-SceI fragment on the vector pTKSCS (Fig. 2A) is amplified by PCR technology, the homologous recombination fragment is transformed into the recombinant strain YH3, and the pyruvate kinase coding gene *pykF* is knocked out by λ-Red homologous recombination technology to obtain the recombinant strain YH4. The recombinant expression vector pACYCDuet-glcK-pgi is transformed into *Escherichia coli* YH4 to obtain recombinant strain YH4-1.

In a specific embodiment, the present disclosure designs primers containing the upstream and downstream homology arms of the phosphoglucomutase coding gene *pgm* of *Escherichia coli.* The SceI-tet-SceI fragment on the vector pTKSCS (Fig. 2A) is amplified by PCR technology, the homologous recombination fragment is transformed into the recombinant strain YH4, and the phosphoglucomutase coding gene *pgm* is knocked out by λ-Red homologous recombination technology to obtain the recombinant strain YH5. The recombinant expression vector pACYCDuet-glcK-pgi is transformed into *Escherichia coli* YH5 to obtain recombinant strain YH5-1.

In a specific embodiment, the present disclosure designs primers containing the upstream and downstream homology arms of the glucose-6-phosphate dehydrogenase coding gene *zwf* of *Escherichia coli.* The SceI-tet-SceI fragment on the vector pTKSCS (Fig. 2A) is amplified by PCR technology, the homologous recombination fragment is transformed into the recombinant strain YH5, and the glucose-6-phosphate dehydrogenase coding gene *zwf* is knocked out by λ-Red homologous recombination technology to obtain the recombinant strain YH6. The recombinant expression vector pACYCDuet-glcK-pgi is transformed into *Escherichia coli* YH6 to obtain recombinant strain YH6-1.

In a specific embodiment, the present disclosure amplifies tagatose-6-phosphate epimerase T6PE gene from *Agrobacterium tumefaciens str. C58* and tagatose-6-phosphate phosphatase T6PP gene derived from *Archaeoglobus fulgidus* and/or *Archaeoglobus profundus,* and constructs them into the expression vector pETDuet to obtain the recombinant expression vector pETDuet-T6PE-T6PP, and the recombinant expression vector pETDuet-T6PE-T6PP is transformed into *Escherichia coli* MG1655 (DE3) to obtain recombinant strain MG1655-1.

In a specific embodiment, the present disclosure constructs the recombinant expression vector pETDuet-T6PE-T6PP carrying the tagatose-6-phosphate epimerase T6PE gene and tagatose-6-phosphate phosphatase T6PP gene into the recombinant strain YH1 to obtain recombinant strain YH1-1.

In a specific embodiment, the present disclosure constructs the recombinant expression vector pETDuet-T6PE-T6PP carrying the tagatose-6-phosphate epimerase T6PE gene and tagatose-6-phosphate phosphatase T6PP gene into the recombinant strain YH2 to obtain recombinant strain YH2-1.

In a specific embodiment, the present disclosure constructs the recombinant expression vector pETDuet-T6PE-T6PP carrying the tagatose-6-phosphate epimerase T6PE gene and tagatose-6-phosphate phosphatase T6PP gene into the recombinant strain YH3-1 to obtain recombinant strain YH3-2.

In a specific embodiment, the present disclosure constructs the recombinant expression vector pETDuet-T6PE-T6PP carrying the tagatose-6-phosphate epimerase T6PE gene and tagatose-6-phosphate phosphatase T6PP gene into the recombinant strain YH4-1 to obtain recombinant strain YH4-2.

In a specific embodiment, the present disclosure constructs the recombinant expression vector pETDuet-T6PE-T6PP carrying the tagatose-6-phosphate epimerase T6PE gene and tagatose-6-phosphate phosphatase T6PP gene into the recombinant strain YH5-1 to obtain recombinant strain YH5-2.

In a specific embodiment, the present disclosure constructs the recombinant expression vector pETDuet-T6PE-T6PP carrying the tagatose-6-phosphate epimerase T6PE gene and tagatose-6-phosphate phosphatase T6PP gene into the recombinant strain YH6-1 to obtain recombinant strain YH6-2.

In some specific embodiments of the present disclosure, recombinant strains BMG1655-1, YH1-1, YH2-1, YH3-2, YH4-2, YH5-2, and YH6-2 can be used as engineered strains of *Escherichia coli* for producing tagatose, to ferment glucose or a mixed medium of glucose and glycerol to produce tagatose.

### Construction method of an engineered strain of bacillus subtilis for producing tagatose

In one technical solution, the present disclosure provides a construction method of an engineered strain of *bacillus subtili* for producing tagatose.

In a specific embodiment, the present disclosure designs primers containing the upstream and downstream homology arms of uracil phosphoribosyltransferase coding gene *upp* of *Bacillus subtilis* 168. The upstream and downstream homology arms of the uracil phosphoribosyltransferase coding gene *upp* are amplified by PCR technology and constructed into the integration vector pSS to obtain recombinant integration vector pSS-upp-FR. The recombinant integration vector pSS-upp-FR is transformed into *Bacillus subtilis* SCK6, and then the recombinant engineered strain YJ8 with knockout of uracil phosphoribosyltransferase conding gene *upp* is obtained by intramolecular homologous recombination technology.

In a specific embodiment, the present disclosure designs primers containing the upstream and downstream homology arms of PTS system glucose-specific transfer protein coding gene *ptsG* of *Bacillus subtilis* 168. The upstream and downstream homology arms of the PTS system glucose-specific transfer protein coding gene *ptsG* are amplified by PCR technology and constructed into the integration vector pSS to obtain recombinant integration vector pSS-ptsG-FR. The recombinant integration vector pSS-ptsG-FR is transformed into *Bacillus subtilis* YJ8, and then the recombinant engineered strain YJ9 with knockout of PTS system glucose-specific transfer protein coding gene *ptsG* is obtained by intramolecular homologous recombination technology.

In a specific embodiment, the present disclosure designs primers containing the upstream and downstream homology arms of HPr kinase coding gene *hprK* of *Bacillus subtilis* 168. The upstream and downstream homology arms of the HPr kinase coding gene *hprK* are amplified by PCR technology and constructed into the integration vector pSS to obtain recombinant integration vector pSS-hprK-FR. The recombinant integration vector pSS-hprK-FR is transformed into *Bacillus subtilis* YJ9, and then the recombinant engineered strain YJ10 with knockout of HPr kinase coding gene *hprK* is obtained by intramolecular homologous recombination technology.

In a specific embodiment, the present disclosure amplifies glucokinase coding gene *glcK* from *Bacillus subtilis* 168 and glucose-6-phosphate isomerase coding gene *pgi* from *Bacillus subtilis* 168, and constructes them into the integration vector pSS-ptsG-FR to obtain recombinant integration vector pSS-ptsG-FR-glcK-pgi. The recombinant integration vector pSS-ptsG-FR-glcK-pgi is transformed into *Bacillus subtilis* YJ10, and then the recombinant engineered strain YJ11 with knockout of glucokinase coding gene *glcK* and glucose-6-phosphate isomerase coding gene *pgi* is obtained by intramolecular homologous recombination technology.

In a specific embodiment, the present disclosure designs primers containing the upstream and downstream homology arms of fructose-6-phosphate kinase coding gene *pfkA* of *Bacillus subtilis* 168. The upstream and downstream homology arms of the fructose-6-phosphate kinase coding gene *pfkA* are amplified by PCR technology and constructed into the integration vector pSS to obtain recombinant integration vector pSS-pfkA-FR. The recombinant integration vector pSS-pfkA-FR is transformed into *Bacillus subtilis* YJ11, and then the recombinant engineered strain YJ12 with knockout of fructose-6-phosphate kinase coding gene *pfkA* is obtained by intramolecular homologous recombination technology.

In a specific embodiment, the present disclosure designs primers containing the upstream and downstream homology arms of phosphoglucomutase coding gene *pgm* of *Bacillus subtilis* 168. The upstream and downstream homology arms of the phosphoglucomutase coding gene *pgm* are amplified by PCR technology and constructed into the integration vector pSS to obtain recombinant integration vector pSS-pgm-FR. The recombinant integration vector pSS-pgm-FR is transformed into *Bacillus subtilis* YJ12, and then the recombinant engineered strain YJ13 with knockout of phosphoglucomutase coding gene *pgm* is obtained by intramolecular homologous recombination technology.

In a specific embodiment, the present disclosure designs primers containing the upstream and downstream homology arms of glucose-6-phosphate dehydrogenase coding gene *zwf* of *Bacillus subtilis* 168. The upstream and downstream homology arms of the glucose-6-phosphate dehydrogenase coding gene *zwf* are amplified by PCR technology and constructed into the integration vector pSS to obtain recombinant integration vector pSS-zwf-FR. The recombinant integration vector pSS-zwf-FR is transformed into *Bacillus subtilis* YJ13, and then the recombinant engineered strain YJ14 with knockout of glucose-6-phosphate dehydrogenase coding gene *zwf* is obtained by intramolecular homologous recombination technology.

In a specific embodiment, the present disclosure amplifies tagatose-6-phosphate epimerase T6PE gene from *Agrobacterium tumefaciens str. C58* and tagatose-6-phosphate phosphatase T6PP gene derived from *Archaeoglobus fulgidus* and/or *Archaeoglobus profundus,* and constructs them into the expression vector pWB980 to obtain the recombinant expression vector pWB980-T6PE-T6PP, and the recombinant expression vector pWB980-T6PE-T6PP is transformed into *Bacillus subtilis* SCK6 to obtain recombinant strain SCK6-1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ8 to obtain recombinant strain YJ8-1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ9 to obtain recombinant strain YJ9-1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ10 to obtain recombinant strain YJ10-1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ11 to obtain recombinant strain YJ11 -1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ12 to obtain recombinant strain YJ12-1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ13 to obtain recombinant strain YJ13-1.

In a specific embodiment, the present disclosure constructs the recombinant expression vector pWB980-T6PE-T6PP carrying the tagatose-6-phosphate epimerase T6PE gene and tagatose-6-phosphate phosphatase T6PP gene into the recombinant strain YJ14 to obtain recombinant strain YJ14-1.

In some specific embodiments of the present disclosure, recombinant strains SCK6-1, YJ8-1, YJ9-1, YJ10-1, YJ11-1, YJ12-1, YJ13-1 and YJ14-1 can be used as engineered strains of *bacillus subtili* for producing tagatose, to ferment glucose or a mixed medium of glucose and glycerol to produce tagatose.

### Tagatose production method

In one technical solution, the present disclosure provides a tagatose production method.

In a specific embodiment, the tagatose production method provided by the present disclosure comprises the steps:
(1) fermenting and culturing tagatose production strain;
(2) after the fermentation reaction is completed, collecting fermentation reaction liquid, and separating tagatose therefrom.

In the above production process, the tagatose production strain can use glucose and glycerol as substrates to efficiently transform and produce tagatose to obtain tagatose with a high yield. The tagatose production method of the present disclosure uses glucose and glycerol as substrates, has wide sources of raw materials which are cheap, does not require a multi-enzyme separation process, has high environmental friendliness, low production cost, and has important process application value.

In some embodiments, the culture medium for fermenting and culturing the engineered strain of *Escherichia coli* for producing tagatose is LB medium (10 g/L peptone, 5 g/L yeast extract and 10 g/L NaCl, kanamycin 25 ng/mL, ampicillin 100 ng/mL) with addition of glucose at a final concentration of 20 g/L.

In some embodiments, the culture medium for fermenting and culturing the engineered strain of *Escherichia coli* for producing tagatose is M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaCl₂, and 2 g/L yeast extract).

In some embodiments, the culture medium for fermenting the engineered strain of *Bacillus subtilis* for producing tagatose is SR medium (15 g/L peptone, 25 g/L yeast extract and 3 g/L K₂HPO₄ kanamycin 25 ng/mL) with addition of glucose at a final concentration of 20 g/L.

In some embodiments, the culture medium for fermenting the engineered strain of *Bacillus subtilis* for producing tagatose is SR medium (15 g/L peptone, 25 g/L yeast extract and 3 g/L K₂HPO₄, kanamycin 25 ng/mL) with addition of glucose at a final concentration of 20 g/L and glycerol at a final concentration of 20 g/L.

In some embodiments, the fermentation culture condition of the tagatose production strain is to culture the tagatose production strain at 37°C and 200 rpm for 12 to 24 hours.

In some embodiments, tagatose can be separated by common methods in the art, including but not limited to: filtration, decolorization, desalting, concentration, crystallization and HPLC detection.

Methods for manipulating microorganisms are known in the art, such as Modern Methods in Molecular Biology (Online ISBN: 9780471142720, John Wiley and Sons, Inc.), Metabolic Engineering of Microorganisms: Methods and Protocols (Qiong Cheng Ed., Springer) and Metabolic Engineering of Systems: Methods and Protocols (Hal S. Alper Ed., Springer) and other publications.

In the present disclosure, the meanings of SEQ ID NO: 1 to SEQ ID NO: 17, SEQ ID NO: 54 to SEQ ID NO: 55 are as follows:
The sequence as set forth in SEQ ID NO: 1 is the amino acid sequence of glucose-specific transfer protein from *Escherichia coli;*
The sequence as set forth in SEQ ID NO: 2 is the nucleotide sequence encoding glucose-specific transfer protein from *Escherichia coli;*
The sequence as set forth in SEQ ID NO: 3 is the amino acid sequence of glucose-specific transfer protein from *Bacillus subtilis;*
The sequence as set forth in SEQ ID NO: 4 is the nucleotide sequence encoding glucose-specific transfer protein from *Bacillus subtilis;*
The sequence as set forth in SEQ ID NO: 5 is the amino acid sequence of tagatose-6-phosphate epimerase from *Agrobacterium tumefaciens*;
The sequence as set forth in SEQ ID NO: 6 is the nucleotide sequence encoding tagatose-6-phosphate epimerase from *Agrobacterium tumefaciens*;
The sequence as set forth in SEQ ID NO: 7 is the amino acid sequence of tagatose-6-phosphate phosphatase from the genus *Archaeoglobus fulgidus*;
The sequence as set forth in SEQ ID NO: 8 is the nucleotide sequence encoding tagatose-6-phosphate phosphatase from the genus *Archaeoglobus fulgidus*;
The sequence as set forth in SEQ ID NO: 9 is the amino acid sequence of glucokinase from *Escherichia coli;*
The sequence as set forth in SEQ ID NO: 10 is the nucleotide sequence encoding glucokinase from *Escherichia coli;*
The sequence as set forth in SEQ ID NO: 11 is the amino acid sequence of glucokinase from *Bacillus subtilis*;
The sequence as set forth in SEQ ID NO: 12 is the nucleotide sequence encoding glucokinase from *Bacillus subtili*;
The sequence as set forth in SEQ ID NO: 13 is the amino acid sequence of glucose-6-phosphate isomerase from *Escherichia coli*;
The sequence as set forth in SEQ ID NO: 14 is the nucleotide sequence encoding glucose-6-phosphate isomerase from *Escherichia coli*
The sequence as set forth in SEQ ID NO: 15 is the amino acid sequence of glucose-6-phosphate isomerase from *Bacillus subtilis*;
The sequence as set forth in SEQ ID NO: 16 is the nucleotide sequence encoding glucose-6-phosphate isomerase from *Bacillus subtilis*;
The sequence as set forth in SEQ ID NO: 17 is the nucleotide sequence of the SceI-tet-SceI fragment;
The sequence as set forth in SEQ ID NO: 54 is the amino acid sequence of tagatose-6-phosphate phosphatase from the genus *Archaeoglobus profundus*;
The sequence as set forth in SEQ ID NO: 55 is the nucleotide sequence encoding tagatose-6-phosphate phosphatase from the genus *Archaeoglobus profundus.*

### EXAMPLES

Other objects, features and advantages of the present disclosure will become apparent from the following detailed description. It should be understood, however, that the detailed description and specific examples, while indicating specific embodiments of the disclosure, are given for illustrative purposes only, since, after reading this detailed description, various changes and modifications within the spirit and scope of the present disclosure will become apparent to those skilled in the art.

The experimental techniques and methods used in this example are conventional technical methods unless otherwise specified, for example, the experimental methods that do not indicate specific conditions in the following examples are usually according to conventional conditions such as those described in Sambrook et al., Molecular Cloning: Laboratory Manual (New York: Cold Spring Harbor Laboratory Press, 1989), or according to the conditions described in the manufacture conditions recommended by the manufacturer. Materials, reagents, etc. used in the examples, unless otherwise specified, can be obtained through formal commercial channels.

### Example 1 Construction of Escherichia coli recombinant strain YH1

(1) According to the PTS system glucose-specific transfer protein coding gene *ptsG* derived from *Escherichia coli* MG1655 in the KEGG database, primers 1 and 2 comprising an upstream 65 bp homologous fragment and a downstream 65 bp homologous fragment of the PTS system glucose-specific transfer protein coding gene *ptsG* were designed. The SceI-tet-SceI fragment (the nucleotide sequence of the SceI-tet-SceI fragment as set forth in SEQ ID NO: 17) on the pTKSCS vector was amplified by PCR to obtain the resistant fragment 1 containing the upstream and downstream homologous fragments of the *ptsG* gene. The specific primer sequences are as follows:
   Primer 1, having a sequence as set forth in SEQ ID NO: 18: CAGGAGCACTCTCAATTATGTTTAAGAATGCATTTGCTAACCTGCAAAAGGTCG GTAAATCGCTGTACGGCCCCAAGGTCCAAACGGTGA
   Primer 2, having a sequence as set forth in SEQ ID NO: 19: 
GCCATCTGGCTGCCTTAGTCTCCCCAACGTCTTACGGATTAGTGGTTACGGATG
TACTCATCCATCAGCGATTTACCGACCTTTTGCAGGTTAGCTTGGCTTCAGGGATGA
GGCGCCATC
(2) Construction of *Escherichia coli* recombinant strain YH1

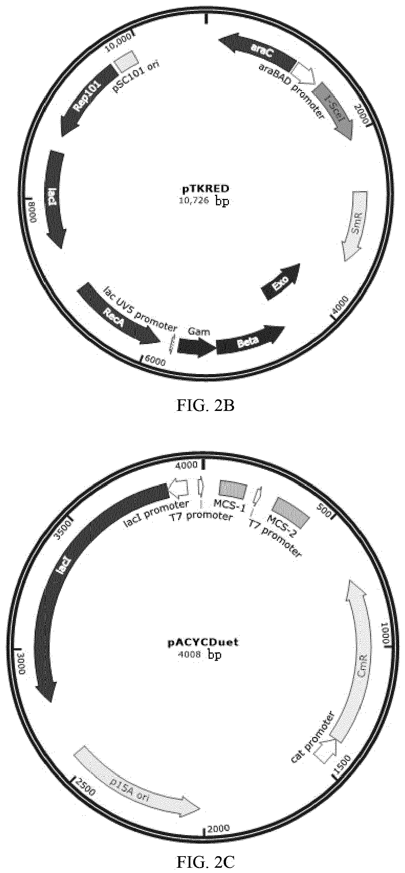

*Escherichia coli* MG1655 (DE3) competent cells (100 uL) were prepared, transferred into the plasmid pTKRed (temperature sensitive plasmid) required for Red recombination (Fig. 2B), coated on a resistant plate containing spectinomycin and incubated overnight at 30°C. A single colony was then picked from the plate and transferred to liquid LB containing spectinomycin and IPTG and incubated at 30°C to prepare MG1655 (DE3) electrotransformed competent cells containing pTKRed (100 uL).

The fragment 1 obtained in step 1 was mixed well with the electrotransformed competent cells, coated on a double antibody plate containing spectinomycin and chloramphenicol after electroporation and incubated at 30°C for 30 hours. Single clones were picked and verified by colony PCR (the verification primers were primer 3 and primer 4). The strain with a PCR product of approximately 1775 bp in size (containing the upstream fragment of the *ptsG* gene on the genome, the SceI-tet-SceI fragment and the downstream fragment of the *ptsG* gene on the genome) was the correct double-crossover strain.

Positive clones were picked and transferred to LB liquid medium containing spectinomycin, IPTG and arabinose for 8 to 12 hours, then the bacterial solution was diluted and coated on a plate containing spectinomycin, IPTG and arabinose for overnight culture. The purpose of this step was to induce the expression of SceI by arabinose, cleave the DNA containing the SceI recognition site, promote intramolecular homologous recombination in positive transformants, and remove the tet-resistant fragment. A number of single clones were picked from the plate and subjected to colony PCR again (the verification primers were primer 3 and primer 4) and transformants with a PCR product of approximately 382 bp of DNA were positive clones. The positive clones were transferred into antibody-free liquid LB, and incubated at 37°C for 8 to 12 hours to remove the pTKRed plasmid. The transformant PCR was subjected to sequencing verfification and the correct strain was saved, that is, the recombinant engineered strain of *Escherichia coli* with the gene encoding PTS system glucose-specific transfer protein knocked out, i.e., the recombinant engineered strain of *Escherichia coli* without enzyme activity of PTS system glucose-specific transfer protein, named for YH1. The specific primer sequences are as follows:
Primer 3, having a sequence as set forth in SEQ ID NO: 20:
   CGTCAAACAAATTGGCACTG
Primer 4, having a sequence as set forth in SEQ ID NO: 21:
   GAACGTCAATAACCTGTTCG

### Example 2 Construction of Escherichia coli recombinant strain YH2

### (1) Construction of recombinant expression vector pACYCDuet-glk-pgi

According to the glucokinase coding gene *glk* and glucose-6-phosphate isomerase coding gene *pgi* derived from *Escherichia coli* in the KEGG database, primers 5 and 6 were designed to amplify *glk,* and primers 7 and 8 were designed to amplify *pgi.* Primers 9 and 10 were designed to amplify the plasmid backbone pACYCDuet (Fig. 2C), and the recombinant expression vector pACYCDuet-glcK was constructed using the simple cloning connection method^{[5]}; then primers 11 and 12 were designed to amplify the plasmid backbone pACYCDuet-glk, the recombinant expression vector pACYCDuet-glk-pgi was obtained by simple cloning method. The specific primer sequences are as follows:
Primer 5, having a sequence as set forth in SEQ ID NO: 22:
   GTTTAACTTTAATAAGGAGATATACCATGACAAAGTATGCATTAGTCGGTG
Primer 6, having a sequence as set forth in SEQ ID NO: 23:
   CGATTACTTTCTGTTCGACTTAAGCATTACAGAATGTGACCTAAGGTCTG
Primer 7, having a sequence as set forth in SEQ ID NO: 24:
   GTTAAGTATAAGAAGGAGATATACATATGAAAAACATCAATCCAACGCAG
Primer 8, having a sequence as set forth in SEQ ID NO: 25:
   TCAGCGGTGGCAGCAGCCTAGGTTAATTAACCGCGCCACGCTTTATAGCG
Primer 9, having a sequence as set forth in SEQ ID NO: 26:
   CAGACCTTAGGTCACATTCTGTAATGCTTAAGTCGAACAGAAAGTAATCG
Primer 10, having a sequence as set forth in SEQ ID NO: 27:
   CACCGACTAATGCATACTTTGTCATGGTATATCTCCTTATTAAAGTTAAAC
Primer 11, having a sequence as set forth in SEQ ID NO: 28:
   GCTATAAAGCGTGGCGCGGTTAATTAACCTAGGCTGCTGCCACCGCTGAG
Primer 12, having a sequence as set forth in SEQ ID NO: 29:
   CTGCGTTGGATTGATGTTTTTCATATGTATATCTCCTTCTTATACTTAAC

### (2) Construction of Escherichia coli recombinant strain YH2

The recombinant expression vector pACYCDuet-glk-pgi was transformed into the recombinant strain YH1 to obtain the recombinant strain YH2.

### Example 3 Construction of Escherichia coli recombinant strain YH3-1

### (1) Construction of pfkA gene knockout recombinant fragment

According to the fructose-6-phosphate kinase coding gene *pfkA* derived from *Escherichia coli* MG1655 in the KEGG database, primers 13 and 14 were designed for the upstream 65 bp homologous fragment and the downstream 65 bp homologous fragment of *pfkA.* The SceI-tet-SceI fragment on the pTKSCS vector was amplified by PCR to obtain the resistant fragment 2 containing the upstream and downstream homologous fragments *of the pfkA* gene. The specific primer sequences are as follows:
Primer 13, having a sequence as set forth in SEQ ID NO: 30: 
CATTCCAAAGTTCAGAGGTAGTCATGATTAAGAAAATCGGTGTGTTGACAAGCGGC
GGTGATGCGtacggccccaaggtccaaacggtga
Primer 14 having a sequence as set forth in SEQ ID NO: 31:

GCCTTTTTCCGAAATCATTAATACAGTTTTTTCGCGCAGTCCAGCCAGTCACCTTTG
AACGGACGCGCATCACCGCCGCTTGTCAACACACCGATttggcttcagggatgaggcgccatc

### (2) Construction of Escherichia coli recombinant strain YH3

*Escherichia coli* YH2 competent cells (100 uL) were prepared, transferred into the plasmid pTKRed (temperature sensitive plasmid) required for Red recombination, coated on a resistant plate containing spectinomycin and incubated overnight at 30°C. A single colony was then picked from the plate and transferred to liquid LB containing spectinomycin and IPTG and incubated at 30°C to prepare YH2 electrotransformed competent cells containing pTKRed (100 uL).

The fragment 2 obtained in step 1 was mixed well with the electrotransformed competent cells, coated on a double antibody plate containing spectinomycin and chloramphenicol after electroporation and incubated at 30°C for 30 hours. Single clones were picked and verified by colony PCR (the verification primers were primer 15 and primer 16). The strain with a PCR product of approximately 1757 bp in size (containing the upstream fragment *of the pfkA* gene on the genome, the SceI-tet-SceI fragment and the downstream fragment *of the pfkA* gene on the genome) was the correct double-crossover strain.

Positive clones were picked and transferred to LB liquid medium containing spectinomycin, IPTG and arabinose for 8 to 12 hours, then the bacterial solution was diluted and coated on a plate containing spectinomycin, IPTG and arabinose for overnight culture. The purpose of this step was to induce the expression of SceI by arabinose, cleave the DNA containing the SceI recognition site, promote intramolecular homologous recombination in positive transformants, and remove the tet-resistant fragment. A number of single clones were picked from the plate and subjected to colony PCR again (the verification primers were primer 15 and primer 16) and transformants with a PCR product of approximately 364 bp of DNA were positive clones. The positive clones were transferred into antibody-free liquid LB, and incubated at 37°C for 8 to 12 hours to remove the pTKRed plasmid. The transformant PCR was subjected to sequencing verfification and the correct strain was saved, that is, the recombinant engineered strain of *Escherichia coli* with fructose 6-phosphate kinase coding gene *pfka* knocked out, i.e., the recombinant engineered strain of *Escherichia coli* without fructose 6-phosphate kinase coding gene *pfka,* named for YH3. The specific primer sequences are as follows:
Primer 15, having a sequence as set forth in SEQ ID NO: 32:
   CATTTGGCCTGACCTGAATC
Primer 16, having a sequence as set forth in SEQ ID NO: 33:
   CGAACGCCTTATCCGGCCTAC

### (3) Construction of Escherichia coli recombinant strain YH3-1

The recombinant expression vector pACYCDuet-glk-pgi was transformed into the recombinant strain YH3 to obtain the recombinant strain YH3-1.

### Example 4 Construction of Escherichia coli recombinant strain YH4-1

### (1) Construction of pykF gene knockout recombinant fragment

According to the pyruvate kinase coding gene *pykF* derived from *Escherichia coli* in the KEGG database, primers 17 and 18 were designed for the upstream 65 bp homologous fragment and the downstream 65 bp homologous fragment of *pykF.* The SceI-tet-SceI fragment on the pTKSCS vector was amplified by PCR to obtain the resistant fragment 3 containing the upstream and downstream homologous fragments of the *pykF* gene. The specific primer sequences are as follows:
Primer 17, having a sequence as set forth in SEQ ID NO: 34: 
AGACTGTCATGAAAAAGACCAAAATTGTTTGCACCATCGGACCGAAAACCGAATCT
GAAGAGATGtacggccccaaggtccaaacggtga
Primer 18, having a sequence as set forth in SEQ ID NO: 35:

CAAAAGCAATATTACAGGACGTGAACAGATGCGGTGTTAGTAGTGCCGCTCGGTAC
CAGTGCACCCATCTCTTCAGATTCGGTTTTCGGTCCGATttggcttcagggatgaggcgccatc

### (2) Construction of Escherichia coli recombinant strain YH4

*Escherichia coli* YH3 competent cells (100 uL) were prepared, transferred into the plasmid pTKRed (temperature sensitive plasmid) required for Red recombination, coated on a resistant plate containing spectinomycin and incubated overnight at 30°C. A single colony was then picked from the plate and transferred to liquid LB containing spectinomycin and IPTG and incubated at 30°C to prepare YH3 electrotransformed competent cells containing pTKRed (100 uL).

The fragment 3 obtained in step 1 was mixed well with the electrotransformed competent cells, coated on a double antibody plate containing spectinomycin and chloramphenicol after electroporation and incubated at 30°C for 30 hours. Single clones were picked and verified by colony PCR (the verification primers were primer 19 and primer 20). The strain with a PCR product of approximately 2310 bp in size (containing the upstream fragment of the *pykF* gene on the genome, the SceI-tet-SceI fragment and the downstream fragment of the *pykF* gene on the genome) was the correct double-crossover strain.

Positive clones were picked and transferred to LB liquid medium containing spectinomycin, IPTG and arabinose for 8 to 12 hours, then the bacterial solution was diluted and coated on a plate containing spectinomycin, IPTG and arabinose for overnight culture. The purpose of this step was to induce the expression of SceI by arabinose, cleave the DNA containing the SceI recognition site, promote intramolecular homologous recombination in positive transformants, and remove the tet-resistant fragment. A number of single clones were picked from the plate and subjected to colony PCR again (the verification primers were primer 15 and primer 16) and transformants with a PCR product of approximately 364 bp of DNA were positive clones. The positive clones were transferred into antibody-free liquid LB, and incubated at 37°C for 8 to 12 hours to remove the pTKRed plasmid. The transformant PCR was subjected to sequencing verfification and the correct strain was saved, that is, the recombinant engineered strain of *Escherichia coli* with pyruvate kinase coding gene *pykF* knocked out, i.e., the recombinant engineered strain of *Escherichia coli* without pyruvate kinase coding gene *pykF,* named for YH4. The specific primer sequences are as follows:
Primer 19, having a sequence as set forth in SEQ ID NO: 36:
   AACTTCGGCACCAGACGTTG
Primer 20, having a sequence as set forth in SEQ ID NO: 37:
   TCTGAACGTCAGAAGACAGC

### (3) Construction of Escherichia coli recombinant strain YH4-1

The recombinant expression vector pACYCDuet-glk-pgi was transformed into the recombinant strain YH4 to obtain the recombinant strain YH4-1.

### Example 5 Construction of Escherichia coli recombinant strain YH5-1

### (1) Construction of pgm gene knockout recombinant fragment

According to the phosphoglucomutase coding gene *pgm* derived from *Escherichia coli* in the KEGG database, primers 21 and 22 were designed for the upstream 65 bp homologous fragment and the downstream 65 bp homologous fragment of *pgm.* The SceI-tet-SceI fragment on the pTKSCS vector was amplified by PCR to obtain the resistant fragment 4 containing the upstream and downstream homologous fragments of the *pgm* gene. The specific primer sequences are as follows:
Primer 21, having a sequence as set forth in SEQ ID NO: 38: 
AAACGTTGCAGACAAAGGACAAAGCAATGGCAATCCACAATCGTGCAGGCCAACC
TGCACAACAGtacggccccaaggtccaaacggtga
Primer 22, having a sequence as set forth in SEQ ID NO: 39:

GTGTTTACGCGTTTTTCAGAACTTCGCTAACAATCTCAACCGCTTCTTTCTCAATCTG
CTTGCGCTGTTGTGCAGGTTGGCCTGCACGATTGTGttggcttcagggatgaggcgccatc

### (2) Construction of Escherichia coli recombinant strain YH5

*Escherichia coli* YH4 competent cells (100 uL) were prepared, transferred into the plasmid pTKRed (temperature sensitive plasmid) required for Red recombination, coated on a resistant plate containing spectinomycin and incubated overnight at 30°C. A single colony was then picked from the plate and transferred to liquid LB containing spectinomycin and IPTG and incubated at 30°C to prepare YH4 electrotransformed competent cells containing pTKRed (100 uL).

The fragment 4 obtained in step 1 was mixed well with the electrotransformed competent cells, coated on a double antibody plate containing spectinomycin and chloramphenicol after electroporation and incubated at 30°C for 30 hours. Single clones were picked and verified by colony PCR (the verification primers were primer 23 and primer 24). The strain with a PCR product of approximately 2200 bp in size (containing the upstream fragment of the *pgm* gene on the genome, the SceI-tet-SceI fragment and the downstream fragment of the *pgm* gene on the genome) was the correct double-crossover strain.

Positive clones were picked and transferred to LB liquid medium containing spectinomycin, IPTG and arabinose for 8 to 12 hours, then the bacterial solution was diluted and coated on a plate containing spectinomycin, IPTG and arabinose for overnight culture. The purpose of this step was to induce the expression of SceI by arabinose, cleave the DNA containing the SceI recognition site, promote intramolecular homologous recombination in positive transformants, and remove the tet-resistant fragment. A number of single clones were picked from the plate and subjected to colony PCR again (the verification primers were primer 15 and primer 16) and transformants with a PCR product of approximately 807 bp of DNA were positive clones. The positive clones were transferred into antibody-free liquid LB, and incubated at 37°C for 8 to 12 hours to remove the pTKRed plasmid. The transformant PCR was subjected to sequencing verfification and the correct strain was saved, that is, the recombinant engineered strain of *Escherichia coli* with phosphoglucomutase coding gene *pgm* knocked out, i.e., the recombinant engineered strain of *Escherichia coli* without phosphoglucomutase coding gene *pgm,* named for YH5. The specific primer sequences are as follows:
Primer 23, having a sequence as set forth in SEQ ID NO: 40:
   CGGTCAAAACGATTAAAGACAAG
Primer 24, having a sequence as set forth in SEQ ID NO: 41:
   CCAGTCGCCAGCTAATGATG

### (3) Construction of Escherichia coli recombinant strain YH5-1

The recombinant expression vector pACYCDuet-glk-pgi was transformed into the recombinant strain YH5 to obtain the recombinant strain YH5-1.

### Example 6 Construction of Escherichia coli recombinant strain YH6-1

### (1) Construction of zwf gene knockout recombinant fragment

According to the glucose-6-phosphate dehydrogenase coding gene *zwf* derived from *Escherichia coli* in the KEGG database, primers 25 and 26 were designed for the upstream 65 bp homologous fragment and the downstream 65 bp homologous fragment of *zwf*. The SceI-tet-SceI fragment on the pTKSCS vector was amplified by PCR to obtain the resistant fragment 5 containing the upstream and downstream homologous fragments of the *zwf* gene. The specific primer sequences are as follows:
Primer 25, having a sequence as set forth in SEQ ID NO: 42: 
GTTAACTTAAGGAGAATGACATGGCGGTAACGCAAACAGCCCAGGCCTGTGACCTG
GTCATTTTCtacggccccaaggtccaaacggtga
Primer 26, having a sequence as set forth in SEQ ID NO: 43:

AAGCGCAGATATTACTCAAACTCATTCCAGGAACGACCATCACGGGTAATCATCGCC
ACCGAGGCGAAAATGACCAGGTCACAGGCCTGGGCTGTttggcttcagggatgaggcgccatc

### (2) Construction of Escherichia coli recombinant strain YH6

*Escherichia coli* YH5 competent cells (100 uL) were prepared, transferred into the plasmid pTKRed (temperature sensitive plasmid) required for Red recombination, coated on a resistant plate containing spectinomycin and incubated overnight at 30°C. A single colony was then picked from the plate and transferred to liquid LB containing spectinomycin and IPTG and incubated at 30°C to prepare YH5 electrotransformed competent cells containing pTKRed (100 uL).

The fragment 5 obtained in step 1 was mixed well with the electrotransformed competent cells, coated on a double antibody plate containing spectinomycin and chloramphenicol after electroporation and incubated at 30°C for 30 hours. Single clones were picked and verified by colony PCR (the verification primers were primer 27 and primer 28). The strain with a PCR product of approximately 2306 bp in size (containing the upstream fragment of the *zwf* gene on the genome, the SceI-tet-SceI fragment and the downstream fragment of the *zwf* gene on the genome) was the correct double-crossover strain.

Positive clones were picked and transferred to LB liquid medium containing spectinomycin, IPTG and arabinose for 8 to 12 hours, then the bacterial solution was diluted and coated on a plate containing spectinomycin, IPTG and arabinose for overnight culture. The purpose of this step was to induce the expression of SceI by arabinose, cleave the DNA containing the SceI recognition site, promote intramolecular homologous recombination in positive transformants, and remove the tet-resistant fragment. A number of single clones were picked from the plate and subjected to colony PCR again (the verification primers were primer 27 and primer 28) and transformants with a PCR product of approximately 913 bp of DNA were positive clones. The positive clones were transferred into antibody-free liquid LB, and incubated at 37°C for 8 to 12 hours to remove the pTKRed plasmid. The transformant PCR was subjected to sequencing verfification and the correct strain was saved, that is, the recombinant engineered strain of *Escherichia coli* with glucose-6-phosphate dehydrogenase coding gene *zwf* knocked out, i.e., the recombinant engineered strain of *Escherichia coli* without glucose-6-phosphate dehydrogenase coding gene *zwf*, named for YH6. The specific primer sequences are as follows:
Primer 27, having a sequence as set forth in SEQ ID NO: 44:
   GATTTGCTCAAATGTTCCAGC
Primer 28, having a sequence as set forth in SEQ ID NO: 45:
   GCAACATGCTTTTCAAAGAG

### (3) Construction of Escherichia coli recombinant strain YH6-1

The recombinant expression vector pACYCDuet-glk-pgi was transformed into the recombinant strain YH6 to obtain the recombinant strain YH6-1.

### Example 7 Construction of Escherichia coli recombinant strain MG1655-1

### (1) Construction of recombinant expression vector pETDuet-t6pe-t6pp

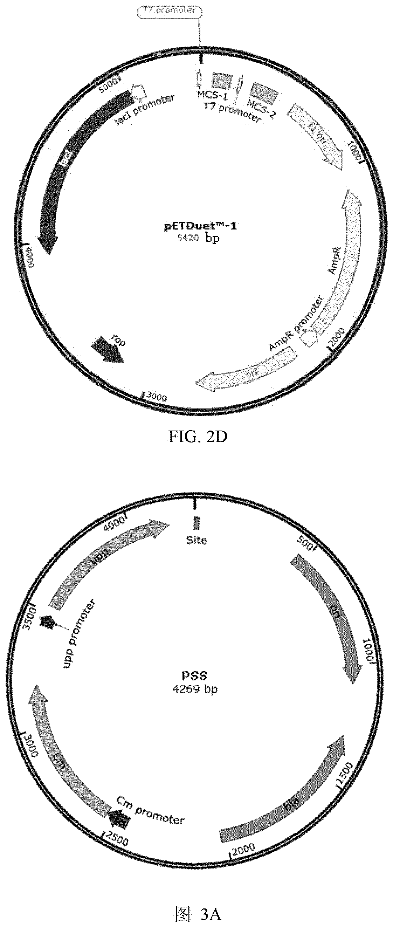

According to the tagatose-6-phosphate epimerase T6PE gene derived from *Agrobacterium tumefaciens str. C58* and tagatose-6-phosphate phosphatase T6PP gene derived from *Archaeoglobus fulgidus* or *Archaeoglobus profundus* in the KEGG database, primers 29 and 30 were designed to amplify t6pe, and primers 31 and 32 were designed to amplify t6pp. Primers 33 and 34 were designed to amplify the plasmid backbone pETDuet (Fig. 2D), and the recombinant expression vector pETDuet-t6pe was constructed using the simple cloning connection method^{[5]}; then primers 35 and 36 were designed to amplify the plasmid backbone pETDuet-t6pe, the recombinant expression vector pETDuet-t6pe-t6pp was obtained by simple cloning method. The specific primer sequences are as follows:
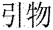 29, having a sequence as set forth in SEQ ID NO: 46:
   GTTAAGTATAAGAAGGAGATATACATATGAACACCGAACATCCGCTGAAAAATG
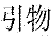 30, having a sequence as set forth in SEQ ID NO: 47:
   CGGTGGCAGCAGCCTAGGTTAATTACTCGAGAATCAGTTTGAATTCACCG
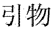 31, having a sequence as set forth in SEQ ID NO: 48:
   GTTTAACTTTAAGAAGGAGATATACCATGTTCAAGCCGAAAGCGATCGCG
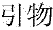 32, having a sequence as set forth in SEQ ID NO: 49:
   GATTACTTTCTGTTCGACTTAAGCATTAACGCAGCAGGCCCAGAAACTGCAG
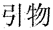 33, having a sequence as set forth in SEQ ID NO: 50:
   CATTTTTCAGCGGATGTTCGGTGTTCATATGTATATCTCCTTCTTATACTTAAC
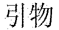 34, having a sequence as set forth in SEQ ID NO: 51:
   CGGTGAATTCAAACTGATTCTCGAGTAATTAACCTAGGCTGCTGCCACCG
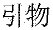 35, having a sequence as set forth in SEQ ID NO: 52:
   CTGCAGTTTCTGGGCCTGCTGCGTTAATGCTTAAGTCGAACAGAAAGTAATC
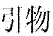 36, having a sequence as set forth in SEQ ID NO: 53:
   CGCGATCGCTTTCGGCTTGAACATGGTATATCTCCTTCTTAAAGTTAAAC

### (2) Construction of Escherichia coli recombinant strain MG1655-1

The recombinant expression vector pETDuet-t6pe-t6pp was transformed into *Escherichia coli* MG1655 (DE3) to obtain the recombinant strain MG1655-1.

In the same way, the recombinant expression vector pETDuet-t6pe-t6pp was introduced into *Escherichia coli* recombinant strains YH1, YH2, YH3-1, YH4-1, YH5-1, YH6-1 to obtain *Escherichia coli* recombinant strains YH1-1, YH2-1, YH3-2, YH4-2, YH5-2, YH6-2 with enhanced enzyme activity of tagatose 6-phosphate epimerase and tagatose 6-phosphate phosphatase.

### Example 8 Application of Escherichia coli recombinant strain MG1655-1 in production of tagatose

### (1) Fermentation of glucose by Escherichia coli recombinant strain MG1655-1 to synthesize tagatose

A 100 mL LB medium (10 g/L peptone, 5 g/L yeast extract and 10 g/L NaCl, kanamycin 25 ng/mL) with a final concentration of 20 g/L glucose was used to incubate *Escherichia coli* recombinant strain MG1655-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 1.0% after 12 hours of fermentation.

### (2) Fermentation of glucose and glycerol by Escherichia coli recombinant strain MG1655-1 to synthesize tagatose

A 100 mL M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaCl₂, and 2 g/L yeast extract) was used to incubate *Escherichia coli* recombinant strain MG1655-1 for 12 to 24 hours at 37°C and 200 pETDuet-t6pe, the recombinant expression vector pETDuet-t6pe-t6pp was obtained by simple cloning method. The specific primer sequences are as follows:
Primer 29, having a sequence as set forth in SEQ ID NO: 46:
   GTTAAGTATAAGAAGGAGATATACATATGAACACCGAACATCCGCTGAAAAATG
Primer 30, having a sequence as set forth in SEQ ID NO: 47:
   CGGTGGCAGCAGCCTAGGTTAATTACTCGAGAATCAGTTTGAATTCACCG
Primer 31, having a sequence as set forth in SEQ ID NO: 48:
   GTTTAACTTTAAGAAGGAGATATACCATGTTCAAGCCGAAAGCGATCGCG
Primer 32, having a sequence as set forth in SEQ ID NO: 49:
   GATTACTTTCTGTTCGACTTAAGCATTAACGCAGCAGGCCCAGAAACTGCAG
Primer 33, having a sequence as set forth in SEQ ID NO: 50:
   CATTTTTCAGCGGATGTTCGGTGTTCATATGTATATCTCCTTCTTATACTTAAC
Primer 34, having a sequence as set forth in SEQ ID NO: 51:
   CGGTGAATTCAAACTGATTCTCGAGTAATTAACCTAGGCTGCTGCCACCG
Primer 35, having a sequence as set forth in SEQ ID NO: 52:
   CTGCAGTTTCTGGGCCTGCTGCGTTAATGCTTAAGTCGAACAGAAAGTAATC
Primer 36, having a sequence as set forth in SEQ ID NO: 53:
   CGCGATCGCTTTCGGCTTGAACATGGTATATCTCCTTCTTAAAGTTAAAC

### (2) Construction of Escherichia coli recombinant strain MG1655-1

The recombinant expression vector pETDuet-t6pe-t6pp was transformed into *Escherichia coli* MG1655 (DE3) to obtain the recombinant strain MG1655-1.

In the same way, the recombinant expression vector pETDuet-t6pe-t6pp was introduced into *Escherichia coli* recombinant strains YH1, YH2, YH3-1, YH4-1, YH5-1, YH6-1 to obtain *Escherichia coli* recombinant strains YH1-1, YH2-1, YH3-2, YH4-2, YH5-2, YH6-2 with enhanced enzyme activity of tagatose 6-phosphate epimerase and tagatose 6-phosphate phosphatase.

### Example 8 Application of Escherichia coli recombinant strain MG1655-1 in production of tagatose

### (1) Fermentation of glucose by Escherichia coli recombinant strain MG1655-1 to synthesize tagatose

A 100 mL LB medium (10 g/L peptone, 5 g/L yeast extract and 10 g/L NaCl, kanamycin 25 ng/mL) with a final concentration of 20 g/L glucose was used to incubate *Escherichia coli* recombinant strain MG1655-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 1.0% after 12 hours of fermentation.

### (2) Fermentation of glucose and glycerol by Escherichia coli recombinant strain MG1655-1 to synthesize tagatose

A 100 mL M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaCl₂, and 2 g/L yeast extract) was used to incubate *Escherichia coli* recombinant strain MG1655-1 for 12 to 24 hours at 37°C and 200 3 g/L KH₂PO₄, 1 g/L NH₄Cl, 1 mM MgSO₄, 0.1 mM CaCl₂, and 2 g/L yeast extract) was used to incubate *Escherichia coli* recombinant strain YH2-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 8.0% after 12 hours of fermentation.

### Example 11 Application of Escherichia coli recombinant strain YH3-2 in production of tagatose

### (1) Fermentation of glucose by Escherichia coli recombinant strain YH3-2 to synthesize tagatose

A 100 mL LB medium (10 g/L peptone, 5 g/L yeast extract and 10 g/L NaCl, kanamycin 25 ng/mL, ampicillin 100 ng/mL) with a final concentration of 20 g/L glucose was used to incubate *Escherichia coli* recombinant strain YH3-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 20% after 12 hours of fermentation.

### (2) Fermentation of glucose and glycerol by Escherichia coli recombinant strain YH3-2 to synthesize tagatose

A 100 mL M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaCl₂, and 2 g/L yeast extract) was used to incubate *Escherichia coli* recombinant strain YH3-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 30% after 12 hours of fermentation.

### Example 12 Application of Escherichia coli recombinant strain YH4-2 in production of tagatose

### (1) Fermentation of glucose by Escherichia coli recombinant strain YH4-2 to synthesize tagatose

A 100 mL LB medium (10 g/L peptone, 5 g/L yeast extract and 10 g/L NaCl, kanamycin 25 ng/mL, ampicillin 100 ng/mL) with a final concentration of 20 g/L glucose was used to incubate *Escherichia coli* recombinant strain YH4-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 16% after 12 hours of fermentation.

### (2) Fermentation of glucose and glycerol by Escherichia coli recombinant strain YH4-2 to synthesize tagatose

A 100 mL M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaClz, and 2 g/L yeast extract) was used to incubate *Escherichia coli* recombinant strain YH4-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 32% after 12 hours of fermentation.

### Example 13 Application of Escherichia coli recombinant strain YH5-2 in production of tagatose

### (1) Fermentation of glucose by Escherichia coli recombinant strain YH5-2 to synthesize tagatose

A 100 mL LB medium (10 g/L peptone, 5 g/L yeast extract and 10 g/L NaCl, kanamycin 25 ng/mL, ampicillin 100 ng/mL) with a final concentration of 20 g/L glucose was used to incubate *Escherichia coli* recombinant strain YH5-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 24% after 12 hours of fermentation.

### (2) Fermentation of glucose and glycerol by Escherichia coli recombinant strain YH5-2 to synthesize tagatose

A 100 mL M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaCl₂, and 2 g/L yeast extract) was used to incubate *Escherichia coli* recombinant strain YH5-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 60% after 12 hours of fermentation.

### Example 14 Application of Escherichia coli recombinant strain YH6-2 in production of tagatose

### (1) Fermentation of glucose by Escherichia coli recombinant strain YH6-2 to synthesize tagatose

A 100 mL LB medium (10 g/L peptone, 5 g/L yeast extract and 10 g/L NaCl, kanamycin 25 ng/mL, ampicillin 100 ng/mL) with a final concentration of 20 g/L glucose was used to incubate *Escherichia coli* recombinant strain YH5-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 41% after 12 hours of fermentation.

### (2) Fermentation of glucose and glycerol by Escherichia coli recombinant strain YH6-2 to synthesize tagatose

A 100 mL M9Y medium (10 g/L glycerol, 20 g/L glucose, 6 g/L Na₂HPO₄, 0.5 g/L NaCl, 3 g/L KH₂PO₄, 1 g/L NH₄Cl, 1 mM MgSO₄, 0.1 mM CaCl₂, and 2 g/L yeast extract) was used to incubate *Escherichia coli* recombinant strain YH6-2 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 79% after 12 hours of fermentation.

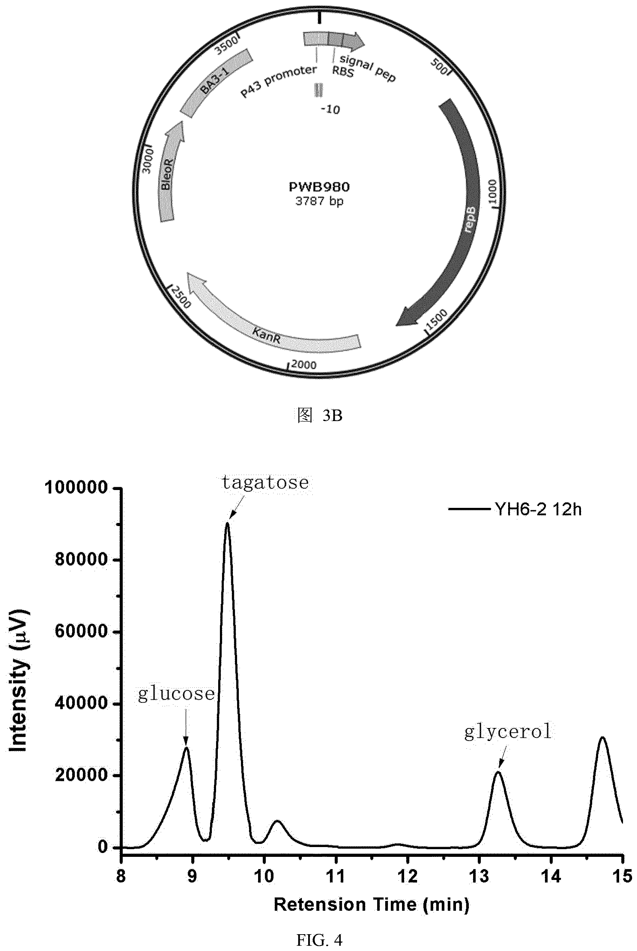

Fig. 4 shows the results of HPLC analysis of fermentation of glucose and glycerol by *Escherichia coli* recombinant strain YH6-2 to produce tagatose. The *Escherichia coli* recombinant strain can simultaneously utilize glucose and glycerol for fermentation to efficiently produce tagatose, and the components in the fermentation reaction liquid are simple, which is easy for the separation and purification of tagatose.

### Example 15 Construction of Bacillus subtilis recombinant strain YJ8

### (1) Construction of recombinant integration vector pSS-upp-FR

According to the gene sequence of uracil phosphoribosyltransferase coding gene *upp* derived from *Bacillus subtilis* 168 in the KEGG database, primers were designed. The upstream 500 bp homologous fragment and the downstream 500 bp homologous fragment of the uracil phosphoribosyltransferase coding gene were amplified by PCR, and constructed into integration vector pSS by simple cloning connection method to obtain a recombinant integration vector pSS-upp-FR.

### (2) Construction of Bacillus subtilis recombinant strain YJ8

*Bacillus subtilis* strain SCK6 super competent cells^{[6]} (200 µL) were prepared, and the recombinant integration vector pSS-upp-FR (1 µg) was evenly mixed with *Bacillus subtilis* strain SCK6 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 1000 bp DNA fragment and 2000 bp DNA fragment obtained by PCR amplification (where, the size of the 1000 bp DNA fragment was the fragment size of the upstream and downstream homology arms of the *upp* coding gene in the vector pSS-upp-FR, the size of the 2000 bp DNA fragment was the size of the fragment on the genome comprising the upstream homology arm of the *upp* conding gene, the *upp* conding gene, and the downstream homology arm of the *upp* conding gene) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain *upp* knockout target transformants by screening and culturing in 5-FU basic salt medium.

Several colonies were picked from the solid plate of 5-FU basic salt medium, and verified by colony PCR again, and the transformants with only 1000 bp DNA fragment obtained by PCR amplification were positive clones. The transformant PCR was subjected to sequencing verfification and the correct strain was saved, that is, the recombinant engineered strain of *Bacillus subtilis* with the gene encoding uracil phosphoribosyltransferase knocked out, i.e., the recombinant engineered strain of *Bacillus subtilis* without uracil phosphoribosyltransferase activity, named YJ8.

### Example 16 Construction of Bacillus subtilis recombinant strain YJ9

### (1) Construction of recombinant integration vector pSS-ptsG-FR

According to the gene sequence of PTS system glucose-specific transfer protein coding gene *ptsG* derived from *Bacillus subtilis* 168 in the KEGG database, primers were designed. The upstream 500 bp homologous fragment and the downstream 500 bp homologous fragment of the PTS system glucose-specific transfer protein coding gene were amplified by PCR, and constructed into integration vector pSS by simple cloning connection method to obtain a recombinant integration vector pSS-ptsG-FR.

### (2) Construction of Bacillus subtilis recombinant strain YJ9

*Bacillus subtilis* strain YJ8 super competent cells (200 µL) were prepared, and the recombinant integration vector pSS-ptsG-FR (1 µg) was evenly mixed with *Bacillus subtilis* strain YJ8 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 1000 bp DNA fragment and 2000 bp DNA fragment obtained by PCR amplification (where, the size of the 1000 bp DNA fragment was the fragment size of the upstream and downstream homology arms of the *ptsG* coding gene in the vector pSS-ptsG-FR, the size of the 2000 bp DNA fragment was the size of the fragment on the genome comprising the upstream homology arm of the *ptsG* conding gene, the *ptsG* conding gene, and the downstream homology arm of the *ptsG* conding gene) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain *ptsG* knockout target transformants by screening and culturing in 5-FU basic salt medium.

Several colonies were picked from the solid plate of 5-FU basic salt medium, and verified by colony PCR again, and the transformants with only 1000 bp DNA fragment obtained by PCR amplification were positive clones. The transformant PCR was subjected to sequencing verfification and the correct strain was saved, that is, the recombinant engineered strain of *Bacillus subtilis* with the gene encoding PTS system glucose-specific transfer protein knocked out, i.e., the recombinant engineered strain of *Bacillus subtilis* without enzyme activity of PTS system glucose-specific transfer protein, named YJ9.

### Example 17 Construction of Bacillus subtilis recombinant strain YJ10

### (1) Construction of recombinant integration vector pSS-hprK-FR

According to the gene sequence of HPr kinase coding gene *hprK* derived from *Bacillus subtilis* 168 in the KEGG database, primers were designed. The upstream 500 bp homologous fragment and the downstream 500 bp homologous fragment of the HPr kinase coding gene were amplified by PCR, and constructed into integration vector pSS by simple cloning connection method to obtain a recombinant integration vector pSS-hprK-FR.

### (2) Construction of Bacillus subtilis recombinant strain YJ10

*Bacillus subtilis* strain YJ9 super competent cells (200 µL) were prepared, and the recombinant integration vector pSS-hprK-FR (1 µg) was evenly mixed with *Bacillus subtilis* strain YJ9 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 1000 bp DNA fragment and 2000 bp DNA fragment obtained by PCR amplification (where, the size of the 1000 bp DNA fragment was the fragment size of the upstream and downstream homology arms of the *hprK* coding gene in the vector pSS-hprK-FR, the size of the 2000 bp DNA fragment was the size of the fragment on the genome comprising the upstream homology arm of the *hprK* conding gene, the *hprK* conding gene, and the downstream homology arm of the *hprK* conding gene) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain *hprK* knockout target transformants by screening and culturing in 5-FU basic salt medium.

Several colonies were picked from the solid plate of 5-FU basic salt medium, and verified by colony PCR again, and the transformants with only 1000 bp DNA fragment obtained by PCR amplification were positive clones. The transformant PCR was subjected to sequencing verfification and the correct strain was saved, that is, the recombinant engineered strain of *Bacillus subtilis* with the gene encoding HPr kinase knocked out, i.e., the recombinant engineered strain of *Bacillus subtilis* without HPr kinase activity, named YJ10.

### Example 18 Construction of Bacillus subtilis recombinant strain YJ11

### (1) Construction of recombinant integration vector pSS-ptsG-FR-glcK-pgi

According to the gene sequence of glucokinase coding gene *glcK* derived from *Bacillus subtilis* 168 and the gene sequence of glucose 6-phosphate isomerase coding gene *pgi* derived from *Bacillus subtilis* 168 in the KEGG database, primers were designed to amplify the *glcK* gene and *pgi* gene, which were constructed into the integration vector pSS-ptsG-FR by simple cloning connection method to obtain a recombinant integration vector pSS-ptsG-FR-glcK-pgi.

### (2) Construction of Bacillus subtilis recombinant strain YJ11

*Bacillus subtilis* strain YJ10 super competent cells (200 µL) were prepared, and the recombinant integration vector pSS-ptsG-FR-glcK-pgi (1 µg) was evenly mixed with *Bacillus subtilis* strain YJ10 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 2000 bp DNA fragment and 4000 bp DNA fragment obtained by PCR amplification (where, the size of the 2000 bp DNA fragment was the size of the fragment in the genome including the upstream homology arm of the *ptsG* conding gene, the *ptsG* conding gene, and the downstream homology arm of the *ptsG* conding gene, and the size of the 4000 bp DNA fragment was the size of the fragment in the vector pSS-ptsG-FR-glcK-pgi comprising the upstream homology arm of the *ptsG* coding gene, the *glcK* gene, the *pgi* gene and the downstream homology arm of *ptsG*) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain target transformant whose *glcK* gene and *pgi* gene were integrated into the *ptsG* gene locus by screening and culturing on 5-FU basic salt medium.

Several colonies were picked from the solid plate of 5-FU basic salt medium, and verified by colony PCR again, and the transformants with only 4000 bp DNA fragment obtained by PCR amplification were positive clones. The transformant PCR was subjected to sequencing verfification and the correct strain was saved, that is, the recombinant engineered strain of *Bacillus subtilis* with the glucokinase coding gene *glcK* gene sequence and the glucose-6-phosphate isomerase coding gene *pgi* integrated into the *ptsG* gene locus, i.e., the recombinant engineered strain of *Bacillus subtilis* with enhanced glucokinase and glucose-6-phosphate isomerase enzyme activities, named YJ11.

### Example 19 Construction of Bacillus subtilis recombinant strain YJ12

### (1) Construction of recombinant integration vector pSS-pfkA-FR

According to the gene sequence of fructose-6-phosphate kinase coding gene *pfkA* derived from *Bacillus subtilis* 168 in the KEGG database, primers were designed. The upstream 500 bp homologous fragment and the downstream 500 bp homologous fragment of the fructose-6-phosphate kinase coding gene were amplified by PCR, and constructed into integration vector pSS by simple cloning connection method to obtain a recombinant integration vector pSS-pfkA-FR.

### (2) Construction of Bacillus subtilis recombinant strain YJ12

*Bacillus subtilis* strain YJ11 super competent cells (200 µL) were prepared, and the recombinant integration vector pSS-pfkA-FR (1 µg) was evenly mixed with *Bacillus subtilis* strain YJ11 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 1000 bp DNA fragment and 2000 bp DNA fragment obtained by PCR amplification (where, the size of the 1000 bp DNA fragment was the fragment size of the upstream and downstream homology arms of the *pfkA* coding gene in the vector pSS-pfkA-FR, the size of the 2000 bp DNA fragment was the size of the fragment on the genome comprising the upstream homology arm of the *pfkA* conding gene, the *pfkA* conding gene, and the downstream homology arm of the *pfkA* conding gene) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain *pfkA* knockout target transformants by screening and culturing in 5-FU basic salt medium.

Several colonies were picked from the solid plate of 5-FU basic salt medium, and verified by colony PCR again, and the transformants with only 1000 bp DNA fragment obtained by PCR amplification were positive clones. The transformant PCR was subjected to sequencing verfification and the correct strain was saved, that is, the recombinant engineered strain of *Bacillus subtilis* with the gene encoding fructose-6-phosphate kinase knocked out, i.e., the recombinant engineered strain of *Bacillus subtilis* without fructose-6-phosphate kinase enzyme activity, named YJ12.

### Example 20 Construction of Bacillus subtilis recombinant strain YJ13

### (1) Construction of recombinant integration vector pSS-pgm-FR

According to the gene sequence of phosphoglucomutase coding gene *pgm* derived from *Bacillus subtilis* 168 in the KEGG database, primers were designed. The upstream 500 bp homologous fragment and the downstream 500 bp homologous fragment of the phosphoglucomutase coding gene were amplified by PCR, and constructed into integration vector pSS by simple cloning connection method to obtain a recombinant integration vector pSS-pgm-FR.

### (2) Construction of Bacillus subtilis recombinant strain YJ13

*Bacillus subtilis* strain YJ12 super competent cells (200 µL) were prepared, and the recombinant integration vector pSS-pgm-FR (1 µg) was evenly mixed with *Bacillus subtilis* strain YJ11 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 1000 bp DNA fragment and 2000 bp DNA fragment obtained by PCR amplification (where, the size of the 1000 bp DNA fragment was the fragment size of the upstream and downstream homology arms of the *pgm* coding gene in the vector pSS-pgm-FR, the size of the 2000 bp DNA fragment was the size of the fragment on the genome comprising the upstream homology arm of the *pgm* conding gene, the *pgm* conding gene, and the downstream homology arm of the *pgm* conding gene) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain *pgm* knockout target transformants by screening and culturing in 5-FU basic salt medium.

Several colonies were picked from the solid plate of 5-FU basic salt medium, and verified by colony PCR again, and the transformants with only 1000 bp DNA fragment obtained by PCR amplification were positive clones. The transformant PCR was subjected to sequencing verfification and the correct strain was saved, that is, the recombinant engineered strain of *Bacillus subtilis* with the gene encoding phosphoglucomutase knocked out, i.e., the recombinant engineered strain of *Bacillus subtilis* without phosphoglucomutase enzyme activity, named YJ13.

### Example 21 Construction of Bacillus subtilis recombinant strain YJ14

### (1) Construction of recombinant integration vector pSS-zwf-FR

According to the gene sequence of glucose-6-phosphate dehydrogenase coding gene *zwf* derived from *Bacillus subtilis* 168 in the KEGG database, primers were designed. The upstream 500 bp homologous fragment and the downstream 500 bp homologous fragment of the glucose-6-phosphate dehydrogenase coding gene were amplified by PCR, and constructed into integration vector pSS by simple cloning connection method to obtain a recombinant integration vector pSS-zwf-FR.

### (2) Construction of Bacillus subtilis recombinant strain YJ14

*Bacillus subtilis* strain YJ13 super competent cells (200 µL) were prepared, and the recombinant integration vector pSS-zwf-FR (1 µg) was evenly mixed with *Bacillus subtilis* strain YJ12 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing chloramphenicol (5 µg/mL), and then placed in a 37°C incubator for 14 to 16 hours.

The positive single-crossover transformant colony grown on the chloramphenicol-resistant plate was picked for colony PCR verification, and those with the two bands of 1000 bp DNA fragment and 2000 bp DNA fragment obtained by PCR amplification (where, the size of the 1000 bp DNA fragment was the fragment size of the upstream and downstream homology arms of the *zwf* coding gene in the vector pSS-pgm-FR, the size of the 2000 bp DNA fragment was the size of the fragment on the genome comprising the upstream homology arm of the *zwf* conding gene, the *zwf* conding gene, and the downstream homology arm of the *zwf* conding gene) were positive clones.

Positive clones were picked and transferred to LB medium without adding antibiotics for 8 to 12 hours, then 200 µL of the bacterial solution was centrifuged to remove the supernatant, then resuspended in sterile water and coated on a solid plate of 5-FU basic salt medium (40% glucose 20.0 ml/L, 4% glutamine 50.0 mL/L, 0.5% histidine 10.0 mL/L, 1% vitamin B1 1.0 mL/L, 20 mM 5-FU 500 µL/L, 10×spizizen 100.0 mL/L, 1000×trace elements 1.0 mL/L), and placed in a 37°C incubator for 24 hours. The purpose of this step was to promote the intramolecular homologous recombination of positive transformants by culturing in LB medium without adding antibiotics, and then to obtain *zwf* knockout target transformants by screening and culturing in 5-FU basic salt medium.

Several colonies were picked from the solid plate of 5-FU basic salt medium, and verified by colony PCR again, and the transformants with only 1000 bp DNA fragment obtained by PCR amplification were positive clones. The transformant PCR was subjected to sequencing verfification and the correct strain was saved, that is, the recombinant engineered strain of *Bacillus subtilis* with the gene encoding glucose-6-phosphate dehydrogenase knocked out, i.e., the recombinant engineered strain of *Bacillus subtilis* without glucose-6-phosphate dehydrogenase enzyme activity, named YJ14.

### Example 22 Construction of Bacillus subtilis recombinant strain SKC6-1

### (1) Construction of recombinant expression vector pWB980-T6PE-T6PP

According to the tagatose-6-phosphate epimerase T6PE gene derived from *Agrobacterium tumefaciens str. C58* and tagatose-6-phosphate phosphatase T6PP gene derived from *Archaeoglobus fulgidus* or *Archaeoglobus profundus* in the KEGG database, primers were designed. Tagatose-6-phosphate epimerase and tagatose-6-phosphate phosphatase gene fragments were obtained by PCR amplification, and constructed into expression vector pWB980 by simple cloning connection method to obtain a recombinant expression vector pWB980-T6PE-T6PP.

### (2) Construction of Bacillus subtilis recombinant strain SCK6-1

*Bacillus subtilis* strain SCK6 super competent cells (200 µL) were prepared, and the recombinant expression vector pWB980-T6PE-T6PP (1 µg) was evenly mixed with *Bacillus subtilis* strain SCK6 super competent cells (200 µL), and then placed in a shaker at 37°C for 90 min to recover. The bacterial solution was coated on solid medium LB (yeast extract 5 g/L, peptone 10 g/L, sodium chloride 10 g/L) containing kanamycin (25 µg/mL), and then placed in a 37°C incubator for 14 to 16 hours. The positive single-crossover transformant colony grown on the kanamycin-resistant plate was picked for colony PCR verification, and the transformants with a 2000 bp DNA fragment obtained by PCR amplification were positive clones. The transformant PCR was subjected to sequencing verfification and the correct strain was saved, that is, the recombinant engineered strain of *Bacillus subtilis* with enhanced enzyme activities of tagatose-6-phosphate epimerase and tagatose-6-phosphate phosphatase, named SCK6-1.

In the same way, the vector pWB980-T6PE-T6PP was introduced into *Bacillus subtilis* recombinant strains YJ8, YJ9, YJ10, YJ11, YJ12, YJ13, YJ14 to obtain *Bacillus subtilis* recombinant strains YJ8-1, YJ9-1, YJ10-1, YJ11-1, YJ12-1, YJ13-1, YJ14-1 with enhanced enzyme activity of tagatose-6-phosphate epimerase and tagatose-6-phosphate phosphatase.

### Example 23 Application of Bacillus subtilis recombinant engineered strain SCK6-1 in production of tagatose

### (1) Fermentation of glucose by Bacillus subtilis recombinant strain SCK6-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain SCK6-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 1.0% after 12 hours of fermentation.

### (2) Fermentation of glucose and glycerol by Bacillus subtilis recombinant strain SCK6-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain SCK6-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 1.0% after 12 hours of fermentation.

### Example 24 Application of Bacillus subtilis recombinant engineered strain YJ8-1 in production of tagatose

### (1) Fermentation of glucose by Bacillus subtilis recombinant strain YJ8-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ8-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 2.0% after 12 hours of fermentation.

### (2) Fermentation of glucose and glycerol by Bacillus subtilis recombinant strain YJ8-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ8-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 2.0% after 12 hours of fermentation.

### Example 25 Application of Bacillus subtilis recombinant engineered strain YJ9-1 in production of tagatose

### (1) Fermentation of glucose by Bacillus subtilis recombinant strain YJ9-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ9-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 2.3% after 12 hours of fermentation.

### (2) Fermentation of glucose and glycerol by Bacillus subtilis recombinant strain YJ9-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ9-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 2.5% after 12 hours of fermentation.

### Example 26 Application of Bacillus subtilis recombinant engineered strain YJ10-1 in production of tagatose

### (1) Fermentation of glucose by Bacillus subtilis recombinant strain YJ10-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ10-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 2.7% after 12 hours of fermentation.

### (2) Fermentation of glucose and glycerol by Bacillus subtilis recombinant strain YJ10-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ10-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 4.0% after 12 hours of fermentation.

### Example 27 Application of Bacillus subtilis recombinant engineered strain YJ11-1 in production of tagatose

### (1) Fermentation of glucose by Bacillus subtilis recombinant strain YJ11-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ11-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 3.0% after 12 hours of fermentation.

### (2) Fermentation of glucose and glycerol by Bacillus subtilis recombinant strain YJ11-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ11-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 5.0% after 12 hours of fermentation.

### Example 28 Application of Bacillus subtilis recombinant engineered strain YJ12-1 in production of tagatose

### (1) Fermentation of glucose by Bacillus subtilis recombinant strain YJ12-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ12-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 8.0% after 12 hours of fermentation.

### (2) Fermentation of glucose and glycerol by Bacillus subtilis recombinant strain YJ12-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ12-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 16.0% after 12 hours of fermentation.

### Example 29 Application of Bacillus subtilis recombinant engineered strain YJ13-1 in production of tagatose

### (1) Fermentation of glucose by Bacillus subtilis recombinant strain YJ13-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ13-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 16% after 12 hours of fermentation.

### (2) Fermentation of glucose and glycerol by Bacillus subtilis recombinant strain YJ13-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ13-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 35% after 12 hours of fermentation.

### Example 30 Application of Bacillus subtilis recombinant engineered strain YJ14-1 in production of tagatose

### (1) Fermentation of glucose by Bacillus subtilis recombinant strain YJ14-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose was used to incubate *Bacillus subtilis* recombinant strain YJ14-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 33% after 12 hours of fermentation.

### (2) Fermentation of glucose and glycerol by Bacillus subtilis recombinant strain YJ14-1 to synthesize tagatose

A 100 mL SR medium (15 g/L peptone, 25 g/L yeast extract and 3 g/L K₂HPO₄, kanamycin 25 ng/mL) with a final concentration of 20 g/L glucose and 20 g/L glycerol was used to incubate *Bacillus subtilis* recombinant strain YJ14-1 for 12 to 24 hours at 37°C and 200 rmp. After fermentation, the sample was centrifuged at 14,000 rmp for 20 min and filtered through a 0.22 µm microporous membrane and the filtrate was subjected to HPLC. HPLC analysis was carried out according to the following conditions: the instrument was Agilent HPLC 1200, analytical column: HPX-87H, mobile phase: 5 mM H₂SO₄, flow rate: 0.6 mL/min, column temperature: 60°C, detector: differential refractive index detector, and the sample volume was 20 µl. The yield of tagatose was 70% after 12 hours of fermentation.

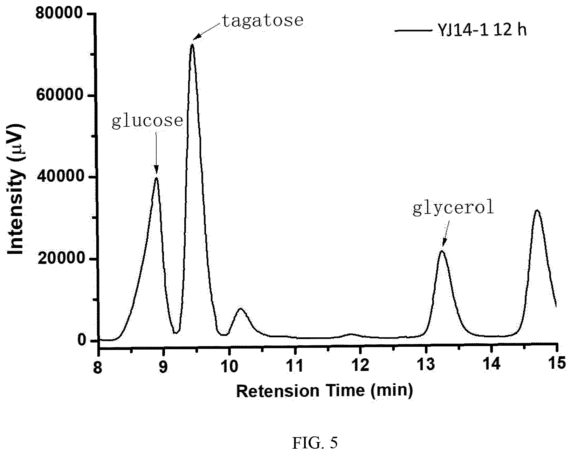

Fig. 5 shows the results of HPLC analysis of fermentation of glucose and glycerol by *Bacillus subtilis* recombinant strain YJ14-1 to produce tagatose. The *Bacillus subtilis* recombinant strain can simultaneously utilize glucose and glycerol for fermentation to efficiently produce tagatose, and the components in the fermentation reaction liquid are simple, which is easy for the separation and purification of tagatose.

You, C., Zhang, X. Z., & Zhang, Y. H. (2012) . Simple cloning via direct transformation of PCR product (DNA Multimer) to Escherichia coli and Bacillus subtilis. Appl. Environ. Microbiol., 78 (5) , 1593-1595. doi:10.1128/AEM.07105-11.

Zhang, X. Z., & Zhang, Y. H. P. (2011). Simple, fast and high-efficiency transformation system for directed evolution of cellulase in Bacillus subtilis. Microb. Biotechnol., 4(1), 98-105. doi:10.1111/j.1751-7915.2010.00230.x.

All technical features disclosed in the present specification can be combined in any combination. Each feature disclosed in the present specification may also be replaced by other features having the same, equivalent or similar functions. Thus, unless stated otherwise, each feature disclosed is only one example of a series of equivalent or similar features.

In addition, from the above description, those skilled in the art can easily understand the key features of the present disclosure from the present disclosure. Many modifications may be made to adapt the invention to various purposes and conditions of use without departing from the spirit and scope of the present disclosure, and such modifications are therefore intended to come within the scope of the appended claims.

## Claims

1. A recombinant microorganism, wherein the recombinant microorganism has at least one of the following characteristics (a)-(c), as compared to a wild-type microorganism:
(a) reduced or lost protein activity of glucose-specific transfer protein of phosphotransferase system and/or expression level of its coding gene;
(b) enhanced enzyme activity of tagatose-6-phosphate epimerase and/or expression level of its coding gene;
(c) enhanced enzyme activity of tagatose-6-phosphate phosphatase and/or expression level of its coding gene.

2. The recombinant microorganism according to claim 1, wherein the glucose-specific transfer protein is selected from the group shown in any one the following (a₁)-(a₃):
(a₁) a polypeptide comprising an amino acid sequence as set forth in SEQ ID NO: 1 or SEQ ID NO: 3 and having glucose-specific transfer protein activity;
(a₂) a polypeptide having an amino acid sequence obtained by substituting, repeating, deleting or adding one or more amino acids to the amino acid sequence as set forth in SEQ ID NO: 1 or SEQ ID NO: 3, and having glucose-specific transfer protein activity;
(a₃) a polypeptide encoded by a nucleotide sequence as set forth in SEQ ID NO: 2 or SEQ ID NO: 4;
optionally, the tagatose-6-phosphate epimerase is selected from the group shown in any of the following (b₁)-(b₃):
(b₁) a polypeptide comprising an amino acid sequence as set forth in SEQ ID NO: 5 and having tagatose-6-phosphate epimerase activity;
(b₂) a polypeptide having an amino acid sequence obtained by substituting, repeating, deleting or adding one or more amino acids to the amino acid sequence as set forth in SEQ ID NO: 5, and having tagatose-6-phosphate epimerase activity;
(b₃) a polypeptide encoded by a nucleotide sequence as set forth in SEQ ID NO: 6;
optionally, the tagatose-6-phosphate phosphatase is selected from the group shown in any of the following (c₁)-(c₃):
(c₁) a polypeptide comprising an amino acid sequence as set forth in SEQ ID NO: 7 or SEQ ID NO: 54 and having tagatose-6-phosphate phosphatase activity;
(c₂) a polypeptide having an amino acid sequence obtained by substituting, repeating, deleting or adding one or more amino acids to the amino acid sequence as set forth in SEQ ID NO: 7 or SEQ ID NO: 54, and having tagatose-6-phosphate phosphatase activity;
(c₃) a polypeptide encoded by a nucleotide sequence as set forth in SEQ ID NO: 8 or SEQ ID NO: 55.

3. The recombinant microorganism according to claim 1 or 2, wherein, as compared to the wild-type microorganism, the recombinant microorganism further has at least one of the following characteristics (d)-(j):
(d) enhanced enzyme activity of glucokinase and/or expression level of its coding gene;
(e) enhanced enzyme activity of glucose-6-phosphate isomerase and/or expression level of its coding gene;
(f) reduced or lost enzyme activity of fructose-6-phosphate kinase and/or expression level of its coding gene;
(g) reduced or lost enzyme activity of pyruvate kinase and/or expression level of its coding gene;
(h) reduced or lost enzyme activity of phosphoglucomutase and/or expression level of its coding gene;
(i) reduced or lost enzyme activity of glucose-6-phosphate dehydrogenase and/or expression level of its coding gene;
(j) reduced or lost enzyme activity of HPr kinase and/or expression level of its coding gene.

4. The recombinant microorganism according to claim 3, wherein the glucokinase is selected from the group shown in any one of the following (d₁)-(d₃):
(d₁) a polypeptide comprising an amino acid sequence as set forth in SEQ ID NO: 9 or SEQ ID NO: 11 and having glucokinase activity;
(d₂) a polypeptide having an amino acid sequence obtained by substituting, repeating, deleting or adding one or more amino acids to the amino acid sequence as set forth in SEQ ID NO:9 or SEQ ID NO: 1 1, and having glucokinase activity;
(d₃) a polypeptide encoded by a nucleotide sequence as set forth in SEQ ID NO: 10 or SEQ ID NO: 12;
optionally, the glucose-6-phosphate isomerase is selected from the group shown in any of the following (e₁)-(e₃):
(e₁) a polypeptide comprising an amino acid sequence as set forth in in SEQ ID NO: 13 or SEQ ID NO: 15 and having glucose-6-phosphate isomerase activity;
(e₂) a polypeptide having an amino acid sequence obtained by substituting, repeating, deleting or adding one or more amino acids to the amino acid sequence as set forth in SEQ ID NO: 13 or SEQ ID NO: 15, and having glucose-6-phosphate isomerase activity;
(e₃) a polypeptide encoded by a nucleotide sequence as set forth in in SEQ ID NO: 14 or SEQ ID NO:16.

5. The recombinant microorganism according to any one of claims 1-4, wherein the recombinant microorganism is derived from *Escherichia coli, Corynebacterium glutamicum, Bacillus subtilis, Lactobacillus* or *Saccharomyces cerevisiae.*

6. A preparation method of the recombinant microorganism according to any one of claims 1-5, wherein the preparation method comprises following steps:
a step of knocking out or knocking down a gene encoding glucose-specific transfer protein of phosphotransferase system in wild-type microorganism;
a step of introducing a recombinant expression vector expressing tagatose-6-phosphate epimerase and tagatose-6-phosphate phosphatase into the recombinant microorganism, or a step of introducing recombinant expression vectors respectively expressing tagatose-6-phosphate epimerase and tagatose-6-phosphate phosphatase into the recombinant microorganism.

7. The preparation method according to claim 6, wherein the preparation method further comprises at least one of following steps:
a step of enhancing expression level of a gene encoding glucokinase in the recombinant microorganism;
a step of enhancing expression level of a gene encoding glucose-6-phosphate isomerase in the recombinant microorganism;
a step of knocking out or knocking down a gene encoding fructose-6-phosphate kinase in the recombinant microorganism;
a step of knocking out or knocking down a gene encoding pyruvate kinase in the recombinant microorganism;
a step of knocking out or knocking down a gene encoding phosphoglucomutase in the recombinant microorganism;
a step of knocking out or knocking down a gene encoding glucose-6-phosphate dehydrogenase in the recombinant microorganism;
a step of knocking out or knocking down a gene encoding HPr kinase in the recombinant microorganism.

8. Use of the recombinant microorganism according to any one of claims 1-5, or a recombinant microorganism prepared by the method according to claim 6 or 7 in production of tagatose.

9. A tagatose production strain, wherein the tagatose production strain is the recombinant microorganism according to any one of claims 1-5, or a recombinant microorganism prepared by the method according to claim 6 or 7.

10. The tagatose production strain according to claim 9, wherein the tagatose production strain uses glucose or glucose and glycerol as substrates;
preferably, the tagatose production strain is derived from *Escherichia coli, Corynebacterium glutamicum, Bacillus subtilis, Lactobacillus* or *Saccharomyces cerevisiae.*

11. A tagatose production method, comprising a step of utilizing the recombinant microorganism according to any one of claims 1-5, a recombinant microorganism prepared by the method according to claim 6 or 7, or the tagatose production strain according to claim 9 or 10 to carry out a fermentation reaction with glucose or glucose and glycerol as substrates.

12. The tagatose production method according to claim 11, further comprising a step of separating tagatose from fermentation reaction liquid after the fermentation reaction is finished.
